# EUROPEAN PATENT APPLICATION

(11) **EP 4 644 417 A1**
(43) Date of publication of application: **05.11.2025**
(21) Application number: 22970280.8
(22) Date of filing: 30.12.2022
(51) Int. Cl.: C07K 16/28, C07K 16/32, A61P 35/00, A61K 39/00

(54) **AFFIBODY BINDING SPECIFICALLY TO CD137 AND USE THEREOF**

(71) Applicant: AbClon Inc., Seoul 08381 (KR)
(72) Inventor: LEE, Hyun-Jong, Incheon 21348 (KR); AHN, Sang-Ho, Seoul 01746 (KR); LEE, Jong-Ho, Anyang-si Gyeonggi-do 13903 (KR); LEE, Jong-Seo, Seongnam-Si Gyeonggi-do 13473 (KR)
(74) Representative: J A Kemp LLP
(86) International application number: PCT/KR2022/021735
(87) International publication number: WO 2024/143643

(57) **Abstract**

The present invention relates to: an affibody binding specifically to the extracellular domain of CD137; a protein complex comprising the described affibody and an antibody that binds specifically to a protein on the surface of cancer cells or an antigen-binding fragment thereof; and a pharmaceutical composition for preventing or treating cancer, comprising same as an active ingredient. By using the affibody binding specifically to CD137, a protein complex binding specifically to T cells and, simultaneously, binding specifically to an antigen on the surface of various types of cancer cells can be developed and effectively used in the prevention or treatment of blood cancer and solid cancer.

## Description

### Technical Field

The present disclosure relates to an affibody binding specifically to CD137 and a use thereof. More specifically, the present disclosure relates to an affibody binding specifically to CD137, a protein complex including the affibody binding specifically to CD137 and an antibody binding specifically to EGFR, HER2, or CD19, and a use thereof for the prevention or treatment of cancer.

### Background Art

In recent years, the following types of immuno-oncology agents have gained attention as promising cancer treatment strategies: immune checkpoint inhibitors such as ipilimumab targeting CTLA-4, pembrolizumab or nivolumab targeting PD-1, and atezolizumab targeting PD-L1; immune cell therapies such as chimeric antigen receptor-T (CART) cells; therapeutic antibodies that bind to antigens on cancer cells, and antibody-drug conjugates (ADCs) that combine such antibodies with drugs; T-cell engaging bispecific antibodies such as blinatumomab, which targets both the CD3 antigen on T cells and the CD19 antigen on B-cell lymphomas, and catumaxomab, which targets both the CD3 antigen on T cells and the epithelial cell adhesion molecule (EpCAM) on cancer cells; and cancer vaccines that deliver tumor-specific antigens or antigens to enhance immune responses.

Among these, T-cell engaging bispecific antibodies such as blinatumomab and catumaxomab are currently the most advanced next-generation immuno-oncology approaches. However, blinatumomab is used only for hematologic cancers and poses safety concerns, and catumaxomab is limited to treating malignant ascites. Therefore, their application in treating a variety of cancers is limited (Lakins MA, et al. Clin Cancer Res. 2020 Aug 1;26(15):4154-4167).

Accordingly, the development of a T-cell engaging bispecific antibody that can be applied not only to hematologic cancers but also to solid tumors is expected to be useful for treating various types of cancers.

Throughout the present specification, numerous references to scientific articles and patent documents are made and cited. The contents disclosed in such references are incorporated herein in their entirety by reference to more clearly describe the level of skill in the art and the technical details of the present disclosure.

### [Prior Art Documents]

Korean Patent No. 10-2017-0012754 A (issued February 3, 2017)
Korean Patent No. 10-2017-0117330 A (issued October 23, 2017)

### Disclosure of Invention

### Technical Problem

Leading to the present disclosure, intensive and thorough research conducted by the present inventors with the aim of developing a T-cell engaging bispecific antibody capable of being applied to both hematologic and solid tumors succeeded in developing an affibody binding specifically to CD137, which is a co-stimulatory molecule the expression of which is increased on the surface of activated T cells, and resulted in the finding that a protein complex developed with the affibody can specifically bind to antigens, such as EGFR, HER2, or CD19, on the surface of cancer cells and thus can serve as a T-cell engaging bispecific antibody capable of being applied to both hematological and solid cancers.

Accordingly, an aspect of the present disclosure is to provide an affibody that specifically binds to the extracellular domain of CD137.

Another aspect of the present disclosure is to provide a protein complex including the affibody described above and an antibody or an antigen-binding fragment thereof that specifically binds to a protein on the surface of a cancer cell.

Yet another aspect of the present disclosure is to provide a pharmaceutical composition for the prevention or treatment of cancer, including the affibody or protein complex described above and a pharmaceutically acceptable carrier.

### Solution to Problem

Provided according to one aspect of the present disclosure is an affibody that specifically binds to the extracellular domain of CD137 and includes an amino acid sequence represented by any one of SEQ ID NOS: 1 to 8.

The present inventors endeavored to develop a T-cell engaging bispecific antibody applicable to both hematologic and solid tumors. As a result, the present inventors developed an affibody that specifically binds to CD137, a co-stimulatory molecule whose expression is increased on the surface of activated T cells, and further developed a protein complex that specifically binds not only to CD137 on activated T cells but also to antigens on the surface of cancer cells such as EGFR, HER2, or CD19. Accordingly, the bispecific antibody comprising the affibody of the present disclosure and an antibody targeting antigens on the surface of cancer cells, i.e., a T-cell engaging bispecific antibody, can be usefully employed in the prevention or treatment of both hematologic and solid cancers.

As used herein, the term "CD137" refers to a co-stimulatory immune checkpoint molecule that is one of the tumor necrosis factor receptor (TNFR) family members, also known as tumor necrosis factor receptor superfamily number 9 (TNFRSF9), 4-1BB, or induced by lymphocyte activation (ILA).

CD137 (4-1BB) is expressed on activated CD8+ T cells, CD4+ T cells, regulatory T cells (Tregs), natural killer T (NKT) cells, natural killer (NK) cells, dendritic cells (DCs), neutrophils, eosinophils, mast cells, and endothelial cells.

Binding of CD137 to 4-1BBL, which is the ligand thereof, induces activation, survival, and effector functions of T cells. The ligand 4-1BBL, a member of the TNF family, is expressed on antigen-presenting cells (APCs) such as mature DCs, activated B cells, and macrophages.

As used herein, the term "affibody" refers to the Z-domain of protein A from *Staphylococcus aureus,* which has affinity for immunoglobulin G (IgG), and is also referred to as "Z body" or "Zb".

An affibody is a small protein composed of 58 amino acid residues. Of these, 13 amino acids that form the IgG binding interface can be varied according to the sequence to allow binding to various target antigens. These randomized sequences can be used to construct libraries. Like antibodies, affibody molecules can be selected from such libraries via screening methods such as phage display or yeast two-hybrid (Y2H) assays to identify binders for specific target antigens.

Furthermore, affibodies are very small in size, with a molecular weight of approximately 6 kDa, and thus can diffuse systemically upon administration and are rapidly cleared through renal filtration, compared to conventional IgG antibodies that have a molecular weight of about 150 kDa. Affibodies have also been developed in the form of bispecific antibodies conjugated to IgGs (Yu F et al., 2014, MAbs). Additionally, affibodies are resistant to heat and alkaline conditions, and can be mass-produced using bacterial systems, resulting in lower production costs than antibodies.

Reference can be made to PCT Publication No. WO95/19374 with respect to an invention relating to first-generation Z-domain-based polypeptide scaffolds and PCT Publication No. WO2009/080811 with respect to an invention relating to second-generation Z-domain-based scaffolds.

As described above, an affibody is a small protein domain that can specifically bind to different target proteins and is designed by randomizing 13 surface residues of the 58-residue IgG Fc-binding Z-domain derived from *Staphylococcus aureus* protein A.

The 13 surface residues subject to such randomization include Q9, Q11, N11, F13, Y14, L17, and H18 located on the first helix, and E24, E25, R27, N28, Q32, and K35 located on the surface of the second helix. The affinity of the wild-type Z-domain for human Fc (IgG) is known to be approximately 10 nM to 60 nM, and protein A is used as an affinity ligand for immunoglobulin capture (see Nord, K., Gunneriusson, E., Ringdahl, J. et al. Nat Biotechnol. 15, 772-777, 1997).

As used herein, the term "AffiMab" refers to a bispecific antibody comprising an affibody molecule fused to either the heavy chain or light chain of an antibody. Due to its modular format, an AffiMab can be easily adapted to various targets (see Volk, AL., Mebrahtu, A., Ko, BK. et al. Drugs R D. 21, 157-168, 2021). The protein complexes, fusion proteins, and bispecific antibodies of the present disclosure may be in the form of AffiMabs.

In an embodiment of the present disclosure, the CD137 is derived from human or cynomolgus monkey.

In an embodiment of the present disclosure, the binding region of the affibody to the extracellular domain of CD137 is located between amino acid residues 64 and 95 of the amino acid sequence of SEQ ID NO: 27. That is, the affibody of the present disclosure specifically binds to amino acid residues 64 to 95 of the extracellular domain of human (*Homo sapiens*) or cynomolgus monkey (*Macaca fascicularis*) CD137.

In another embodiment of the present disclosure, the affibody that specifically binds to the extracellular domain of CD137 may be in the form of an affibody homodimer. The homodimer refers to a protein complex formed by interaction of identical proteins. The affibody homodimer may be in a multimeric form in which affibody monomers are linked to each other. The affibody homodimer may be in a multimeric form in which affibody monomers are connected via amino acid linkers. The affibody homodimer may be implemented as a fusion protein or a conjugate. The affibody homodimer may be linked directly, for example, by known organic chemical methods, or indirectly, for example, via amino acid linkers.

In another embodiment of the present disclosure, the affibody that specifically binds to the extracellular domain of CD137 may be in the form of a complex linked to gold nanoparticles. In such a case, it can be used in nanoparticle-based immunoassays such as ELISA or immuno-PCR to detect CD137-expressing antigen-presenting cells (APCs), such as activated T cells, with high sensitivity and specificity.

In another embodiment of the present disclosure, the affibody that specifically binds to the extracellular domain of CD137 may be in the form of a complex linked to a fluorescent protein. When the affibody of the present disclosure is in the form of a complex with a fluorescent protein, it can be used for specific targeting and imaging of antigen-presenting cells expressing CD137.

Another aspect of the present disclosure provides a nucleic acid molecule including a nucleotide sequence encoding an affibody according to an embodiment of the present disclosure.

In an embodiment of the present disclosure, the nucleic acid molecule includes a nucleotide sequence encoding an affibody that specifically binds to the extracellular domain of CD137.

In an embodiment of the present disclosure, the nucleic acid molecule includes a nucleotide sequence encoding an affibody that specifically binds to the extracellular domain of CD137 which comprises the amino acid sequence of SEQ ID NO: 27.

In an embodiment of the present disclosure, the nucleic acid molecule comprises a nucleotide sequence encoding an affibody that specifically binds to amino acid residues 64 to 95 of the amino acid sequence of SEQ ID NO: 27.

As used herein, the term "nucleic acid molecule" is intended to comprehensively encompass DNA (gDNA and cDNA) and RNA molecules. The term "nucleotide," as used in the nucleic acid molecule, includes not only naturally occurring nucleotides but also analogs in which the sugar or base moiety is modified (see Scheit, Nucleotide Analogs, John Wiley, New York (1980); Uhlman and Peyman, Chemical Reviews, 90:543-584 (1990)).

The nucleotide sequence encoding the affibody of the present disclosure is sufficient as long as it encodes the amino acid sequence constituting the affibody, and it is evident to a person skilled in the art that the nucleotide sequence is not limited to a particular nucleotide sequence.

This is because even if a variation occurs in the nucleotide sequence, the resulting protein may not exhibit any change in the amino acid sequence. This phenomenon is known as codon degeneracy. Accordingly, the nucleotide sequence may comprise codons functionally equivalent to those encoding the same amino acid (for example, due to codon degeneracy, there are six codons for arginine or serine), or codons encoding biologically equivalent amino acids.

In consideration of such biologically equivalent variants, the nucleic acid molecule of the present disclosure that encodes the amino acid sequence constituting the affibody is also construed to include sequences that exhibit substantial identity thereto.

As used herein, the term "substantial identity" refers to a sequence having at least 60% homology (e.g., 61%, 62%, 63%, 64%, 65%, 66%, 67%, 68%, or 69%), more specifically at least 70% homology (e.g., 71%, 72%, 73%, 74%, 75%, 76%, 77%, 78%, or 79%), even more specifically at least 80% homology (e.g., 81%, 82%, 83%, 84%, 85%, 86%, 87%, 88%, or 89%), still more specifically at least 90% homology (e.g., 91%, 92%, 93%, 94%, 95%, 96%, 97%, 98%, or 99%), and most specifically at least 95% homology (e.g., 95%, 96%, 97%, 98%, or 99%) when aligned with the sequence of the present disclosure using an alignment algorithm commonly used in the art. All integers and fractional values between 60% and 100% homology fall within the scope of the present disclosure.

Sequence alignment methods for sequence comparison are known in the art. For alignment methods and algorithms, reference may be made to various literatures, including: Smith and Waterman, Adv. Appl. Math. 2:482 (1981); Needleman and Wunsch, J. Mol. Biol. 48:443 (1970); Pearson and Lipman, Methods in Mol. Biol. 24:307-31 (1988); Higgins and Sharp, Gene 73:237-44 (1988); Higgins and Sharp, CABIOS 5:151-3 (1989); Corpet et al., Nuc. Acids Res. 16:10881-90 (1988); Huang et al., Comp. Appl. BioSci. 8:155-65 (1992); and Pearson et al., Meth. Mol. Biol. 24:307-31 (1994). The NCBI Basic Local Alignment Search Tool (BLAST) (Altschul et al., J. Mol. Biol. 215:403-10 (1990)) is accessible via the National Center for Biotechnology Information (NCBI) and can be used in conjunction with online sequence analysis tools such as blastp, blastn, blastx, tblastn, and tblastx on the internet. BLAST is accessible through the BLAST page on the NCBI website. Instructions for using BLAST can be found on the BLAST Help page of the NCBI website.

According to a specific embodiment of the present disclosure, the polypeptide constituting the affibody that binds to CD137 and the nucleotide sequence encoding the same are included in the sequence listing attached to the present specification.

In an embodiment of the present disclosure, the nucleotide sequence includes a nucleotide sequence selected from SEQ ID NOS: 9 to 16.

Provided according to another aspect of the present disclosure is a recombinant vector including the above-described nucleic acid molecule.

Specifically, the present disclosure provides a recombinant vector carrying a nucleic acid molecule including a nucleotide sequence coding for an affibody that specifically binds to CD137.

In an embodiment of the present disclosure, the recombinant vector is a vector for expressing an affibody that specifically binds to CD137 and includes the above-described nucleic acid molecule.

In an embodiment of the present disclosure, the recombinant vector is a vector for expressing an affibody that specifically binds to the extracellular domain of CD137 and includes the above-described nucleic acid molecule.

In an embodiment of the present disclosure, the recombinant vector is a vector for expressing an affibody that specifically binds to the extracellular domain of CD137 and includes the amino acid sequence of SEQ ID NO: 27.

In an embodiment of the present disclosure, the recombinant vector is a vector for expressing an affibody that specifically binds to amino acid residues 64 to 95 of the amino acid sequence of SEQ ID NO: 27 and includes the above-described nucleic acid molecule.

As used herein, the term "vector" refers to a means for expressing a gene of interest in a host cell, and includes, but is not limited to, phagemid vectors, plasmid vectors, cosmid vectors, bacteriophage vectors, and viral vectors such as adenovirus vectors, retrovirus vectors, and adeno-associated virus vectors.

In an embodiment of the present disclosure, the nucleic acid molecule encoding the affibody is operatively linked to a promoter in the vector of the present disclosure.

As used herein, the term "operatively linked" refers to a functional linkage between a nucleic acid expression control sequence (e.g., a promoter, signal sequence, or an array of transcription factor binding sites) and another nucleic acid sequence, whereby the control sequence regulates the transcription and/or translation of the other nucleic acid sequence.

The recombinant vector system of the present disclosure can be constructed using various methods well known in the art. For specific construction methods, reference may be made to Molecular Cloning, A Laboratory Manual by Sambrook et al., Cold Spring Harbor Laboratory Press (2001), which is incorporated herein by reference.

The vector of the present disclosure can be constructed as a cloning vector or an expression vector. The vector can also be constructed using a prokaryotic or eukaryotic host cell.

For example, when the vector of the present disclosure is an expression vector and the host is a eukaryotic cell, promoters derived from mammalian genomes (e.g., metallothionein promoter, beta-actin promoter, human hemoglobin promoter, and human muscle creatine promoter) or from mammalian viruses (e.g., adenovirus late promoter, vaccinia virus 7.5K promoter, SV40 promoter, cytomegalovirus promoter, HSV tk promoter, MMTV promoter, HIV LTR promoter, Moloney virus promoter, Epstein-Barr virus (EBV) promoter, and Rous sarcoma virus (RSV) promoter) can be used. These vectors generally include a polyadenylation sequence as a transcription termination sequence.

The vector of the present disclosure may be fused with other sequences to facilitate purification of the expressed affibody. Examples of fusion partners include glutathione S-transferase (Pharmacia, USA), maltose-binding protein (NEB, USA), FLAG (IBI, USA), and 6xHis (hexahistidine; Qiagen, USA).

The expression vector of the present disclosure typically includes a selection marker, such as an antibiotic resistance gene commonly used in the art. Examples include resistance genes to ampicillin, gentamicin, carbenicillin, chloramphenicol, streptomycin, kanamycin, geneticin, neomycin, and tetracycline.

Optionally, the vector may additionally carry a gene encoding a reporter molecule (e.g., luciferase or β-glucuronidase).

According to an embodiment of the present disclosure, the expression vector is a vector in which a nucleic acid molecule coding for an affibody binding specifically to human or cynomolgus monkey CD137 is inserted and which includes a promoter operatively linked to the nucleotide sequence of the nucleic acid molecule and adapted to form an RNA molecule in a host cell, and a poly(A) signal sequence for inducing polyadenylation at the 3' end of the RNA molecule in the host cell.

So long as it is well known in the art to enable the stable and continuous cloning and expression of the vector of the present disclosure, any host cell may be available. Suitable eukaryotic host cells include, but are not limited to, *Saccharomyces cerevisiae* (yeast), insect cells, COS7 (monkey kidney cells), NS0 cells, SP2/0 cells, Chinese hamster ovary (CHO) cells, W138 cells, baby hamster kidney (BHK) cells, MDCK cells, myeloma cell lines, HuT 78 cells, and HEK-293 cells, with no limitations thereto.

Provided according to yet another aspect of the present disclosure is an isolated host cell including the above-described recombinant vector.

In an embodiment of the present disclosure, the isolated host cell is a host cell transformed with the recombinant vector of the present disclosure.

As used herein, the terms "transformed," "transduced," "transfected," refer to the process by which exogenous nucleic acid is delivered or introduced into a host cell. A "transformed," "transduced", or "transfected" cell refers to a cell into which an exogenous nucleic acid has been introduced by transformation, transduction, or transfection, and encompasses both the originally modified cell and progeny thereof derived through subsequent culturing.

When the host cell is a eukaryotic cell, methods for introducing the vector of the present disclosure into a host cell may include microinjection (Capecchi, M.R., Cell, 22:479 (1980)), calcium phosphate precipitation (Graham, F.L. et al., Virology, 52:456 (1973)), electroporation (Neumann, E. et al., EMBO J., 1:841

(1982)), liposome-mediated transfection (Wong, T.K. et al., Gene, 10:87 (1980)), DEAE-dextran treatment (Gopal, Mol. Cell Biol., 5:1188-1190 (1985)), and gene bombardment (Yang et al., Proc. Natl. Acad. Sci., 87:9568-9572 (1990)).

The recombinant vector introduced into the host cell according to the present disclosure may express the recombinant affibody within the host cell, allowing for the production of large quantities of the affibody. For example, if the expression vector includes a galactose-inducible promoter, gene expression can be induced in the host cell by treatment with IPTG.

The culturing is generally performed under aerobic conditions such as shaking incubation or rotation using a rotary shaker. The culture temperature is preferably in the range of 10°C to 40°C, and the culturing period is typically from 5 hours to 7 days. The pH of the medium is preferably maintained in the range of 3.0 to 9.0 during culturing. The pH may be adjusted using inorganic or organic acids, alkaline solutions, urea, calcium carbonate, ammonia, or the like. During culturing, antibiotics such as ampicillin, streptomycin, chloramphenicol, kanamycin, and tetracycline may be added if necessary to maintain and express the recombinant vector. When culturing a host cell transformed with a recombinant expression vector containing an inducible promoter, an appropriate inducer may be added to the medium if needed. For example, when the expression vector contains a lac promoter or lactose-inducible promoter, isopropyl-β-D-thiogalactopyranoside (IPTG) may be added; when the vector contains a trp promoter, indoleacrylic acid may be added to the medium.

Yet another aspect of the present disclosure provides a pharmaceutical composition for the prevention or treatment of cancer, including the affibody according to an embodiment of the present disclosure and a pharmaceutically acceptable carrier.

Since the above-described affibody specifically binds to cells expressing CD137, particularly activated CD8⁺ T cells, dendritic cells, and NK cells, the affibody may act as an agonist to CD137, thereby activating T cells and inducing NK cell-mediated antibody-dependent cell-mediated cytotoxicity (ADCC) or T-cell-mediated cytotoxicity, and thus can be usefully employed in the prevention or treatment of cancer.

Because the affibody specifically binding to CD137 is used as an active ingredient in the pharmaceutical composition of the present disclosure, descriptions common to both have been omitted herein to avoid undue complexity in the present specification.

In an embodiment of the present disclosure, the cancer is a hematologic cancer or a solid tumor.

In a specific embodiment of the present disclosure, the solid tumor may be selected from the group consisting of brain tumors, benign astrocytoma, malignant astrocytoma, pituitary adenoma, meningioma, brain lymphoma, oligodendroglioma, ependymoma, brainstem tumor, head and neck tumors, laryngeal cancer, oropharyngeal cancer, nasal cavity cancer, paranasal sinus cancer, nasopharyngeal cancer, salivary gland cancer, hypopharyngeal cancer, thyroid cancer, oral cancer, thoracic tumors, small cell lung cancer, non-small cell lung cancer, thymic cancer, mediastinal tumor, esophageal cancer, breast cancer, male breast cancer, abdominal tumors, gastric cancer, liver cancer, gallbladder cancer, bile duct cancer, pancreatic cancer, small intestine cancer, colorectal cancer, rectal cancer, anal cancer, bladder cancer, kidney cancer, male genital tumors, penile cancer, prostate cancer, female genital tumors, cervical cancer, endometrial cancer, ovarian cancer, uterine sarcoma, vaginal cancer, vulvar cancer, female urethral cancer, and skin cancer, but with no limitations thereto.

In a specific embodiment of the present disclosure, the hematologic cancer may be selected from the group consisting of acute myeloid leukemia, chronic myeloid leukemia, myelodysplastic syndrome, myeloproliferative neoplasm, polycythemia vera, essential thrombocythemia, myelofibrosis, monocytic leukemia, erythroleukemia, megaloblastic leukemia, basophilic leukemia, eosinophilic leukemia, acute lymphoblastic leukemia, chronic lymphocytic leukemia, multiple myeloma, lymphoma, B-cell lymphoma, marginal zone B-cell lymphoma, Hodgkin lymphoma, non-Hodgkin lymphoma, diffuse large B-cell lymphoma, extranodal marginal zone lymphoma, extranodal NK/T-cell lymphoma, peripheral T-cell lymphoma, Burkitt lymphoma, precursor cell tumors, mantle cell lymphoma, follicular lymphoma, immunodeficiency-associated lymphoproliferative disorder, nodular lymphocyte predominant Hodgkin disease, nodular sclerosis classical Hodgkin disease, lymphocyte-rich classical Hodgkin disease, mixed cellularity classical Hodgkin disease, lymphocyte-depleted classical Hodgkin disease, and chemotherapy-resistant prolymphocytic leukemia, but is not limited thereto.

The pharmaceutically acceptable carrier included in the pharmaceutical composition of the present disclosure may be any conventionally used carrier, and examples thereof include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylparaben, propylparaben, talc, magnesium stearate, and mineral oil, but are not limited thereto. The pharmaceutical composition of the present disclosure may further comprise lubricants, wetting agents, sweeteners, flavoring agents, emulsifiers, suspending agents, and preservatives. Suitable pharmaceutically acceptable carriers and formulations are described in detail in Remington's Pharmaceutical Sciences, 19th edition, 1995.

The pharmaceutical composition of the present disclosure may be administered orally or non-orally, for example, via intravenous injection, subcutaneous injection, intramuscular injection, intraperitoneal injection, intrastermal injection, topical administration, intranasal administration, pulmonary administration, or rectal administration.

The appropriate dosage of the pharmaceutical composition of the present disclosure may vary depending on formulation method, mode of administration, age, weight, sex, pathological condition, diet, time of administration, route of administration, rate of excretion, and responsiveness, and a person skilled in the art can readily determine and prescribe an effective amount for the desired therapeutic or prophylactic effect. According to a preferred embodiment of the present disclosure, the daily dose of the pharmaceutical composition is 0.0001 to 100 mg/kg. As used herein, the term "pharmaceutically effective amount" refers to an amount sufficient to prevent or treat the aforementioned disease.

As used herein, the term "prevention" refers to prophylactic or protective treatment or the inhibition of a disease or disease state. The term "treatment" refers to the reduction, suppression, alleviation, or eradication of a disease state.

The pharmaceutical composition of the present disclosure may be formulated into unit dosage forms or placed in multi-dose containers by using a pharmaceutically acceptable carrier and/or excipient, according to methods easily implementable by those skilled in the art. The formulation may be in the form of a solution, suspension, or emulsion in an oil or aqueous medium, or may be in the form of an extract, powder, suppository, granule, tablet, or capsule, and may further include dispersing agents or stabilizers.

Provided according to one aspect of the present disclosure is a protein complex including: an affibody that specifically binds to the extracellular domain of CD137 according to an embodiment of the present disclosure; and an antibody or antigen-binding fragment thereof that specifically binds to a protein on the surface of a cancer cell.

As used herein, the term "protein complex" refers to a group of at least two associated polypeptide chains.

As used herein, the term "protein on the surface of a cancer cell" refers to a protein that is embedded in or spans the cell membrane. The protein on the surface of a cancer cell may be, but is not limited to, an integral membrane protein, transmembrane protein, lipid-anchored membrane protein, peripheral membrane protein, antigen, proteoglycan, or mucin that is present or expressed on the surface of a cancer cell.

The protein on the surface of a cancer cell may be a protein overexpressed relative to normal cells. It may also be a mutated form of a wild-type protein. The protein may be a cancer cell-specific protein or a tumor marker. The tumor marker may be any protein present in or produced by cancer cells or other cells as a result of cancer or specific noncancerous benign conditions.

In an embodiment of the present disclosure, the protein complex is in the form of a multimer in which each monomer of the affibody and each monomer of the antibody or antigen-binding fragment that specifically binds to a protein on the surface of a cancer cell are connected.

In an embodiment of the present disclosure, the protein complex may be in the form of a multimer in which the affibody and each antibody or antigen-binding fragment thereof are covalently linked.

In an embodiment of the present disclosure, the protein complex may be in the form of a multimer in which the affibody and each antibody or antigen-binding fragment thereof are connected via an amino acid linker.

In a specific embodiment of the present disclosure, the protein complex may be implemented in the form of a fused protein or conjugate.

Accordingly, the affibody and the antibody or antigen-binding fragment thereof may be linked either chemically (via known organic chemical methods) or by other means (e.g., expressed as a fusion protein or indirectly linked via a linker such as an amino acid linker) .

According to a specific embodiment of the present disclosure, each monomer constituting the protein complex is connected by at least one linker. In this case, the linker may be an amino acid sequence represented by the general formula (GnSm)p or (SmGn)p:
wherein n, m, and p are independently determined:
n is an integer of 1 to 7;
m is an integer of 0 to 7;
with the sum of n and m being an integer of 8 or less; and
p is an integer of 1 to 7.

In another specific embodiment of the present disclosure, n is an integer of 1 to 5 and m is an integer of 0 to 5. In a more specific embodiment, n = 4 and m = 1. In an even more specific embodiment, the linker is (GGGGS)₃. In another embodiment, the linker is GGGGS. In yet another specific embodiment, the linker is VDGS. In another specific embodiment, the linker is ASGS.

In an embodiment of the present disclosure, the protein complex may be a multi-specific antibody targeting two or more targets.

In an embodiment of the present disclosure, the protein complex may be a monoclonal antibody, polyclonal antibody, scFv, Fab, F(ab), F(ab)₂, scFv-Fc, minibody, diabody, triabody, tetrabody, bispecific antibody, multispecific antibody, human antibody, humanized antibody, chimeric antibody, chemobody, optobody, or an antigen-binding fragment thereof.

In a specific embodiment of the present disclosure, the protein complex is a bispecific antibody. In another specific embodiment of the present disclosure, the protein complex is a tetravalent antibody.

In a specific embodiment of the present disclosure, the protein complex is a trispecific antibody.

In a specific embodiment of the present disclosure, the protein complex is a T-cell engaging bispecific antibody.

In another specific embodiment of the present disclosure, the protein complex is a monoclonal bispecific antibody.

In yet another specific embodiment of the present disclosure, the protein complex is a polyclonal bispecific antibody.

In a specific embodiment of the present disclosure, the antibody or antigen-binding fragment thereof that specifically binds to the protein on the surface of a cancer cell may be a monoclonal antibody, polyclonal antibody, scFv, Fab, F(ab), F(ab)₂, scFv-Fc, minibody, diabody, triabody, tetrabody, bispecific antibody, trispecific antibody, multispecific antibody, bivalent antibody, tetravalent antibody, human antibody, humanized antibody, chimeric antibody, or an antigen-binding fragment thereof.

In an embodiment of the present disclosure, the affibody is fused to the heavy chain or light chain of the antibody or antigen-binding fragment thereof.

In a specific embodiment of the present disclosure, the affibody is fused to the C-terminus of the heavy chain or light chain of the antibody or antigen-binding fragment thereof.

In another specific embodiment of the present disclosure, the affibody is fused to the N-terminus of the heavy chain or light chain of the antibody or antigen-binding fragment thereof.

In yet another specific embodiment of the present disclosure, the affibody is fused to both the C-terminus and N-terminus of the heavy chain or light chain of the antibody or antigen-binding fragment thereof.

In an embodiment of the present disclosure, the protein on the surface of a cancer cell is selected from epidermal growth factor receptor (EGFR), human epidermal growth factor receptor 2 (HER2), or cluster of differentiation 19 (CD19).

In a specific embodiment of the present disclosure, the antibody or antigen-binding fragment that specifically binds to EGFR includes:
(a) cetuximab; or
(b) a heavy chain variable region including CDR-H1 having the amino acid sequence of SEQ ID NO: 17, CDR-H2 having the amino acid sequence of SEQ ID NO: 18, and CDR-H3 having the amino acid sequence of SEQ ID NO: 19; and a light chain variable region including CDR-L1 having the amino acid sequence of SEQ ID NO: 20, CDR-L2 having the amino acid sequence of SEQ ID NO: 21, and CDR-L3 having the amino acid sequence of SEQ ID NO: 22.

Cetuximab is a commercially available antibody that specifically binds to human EGFR, and it will be understood by those skilled in the art that any antibody or antigen-binding fragment thereof known to specifically bind to human EGFR may be used without limitation.

In a specific embodiment of the present disclosure, the protein complex includes: an affibody that specifically binds to the extracellular domain of CD137 according to an embodiment of the present disclosure; and cetuximab.

More specifically, in a specific embodiment of the present disclosure, the protein complex includes: an affibody specifically binding to the extracellular domain of CD137 and including an amino acid sequence represented by any one of SEQ ID NOS: 1 to 8; and cetuximab.

In another specific embodiment of the present disclosure, the protein complex includes: an affibody that specifically binds to the extracellular domain of CD137; and
an antibody or antigen-binding fragment thereof including a heavy chain variable region including CDR-H1 having the amino acid sequence of SEQ ID NO: 17, CDR-H2 having the amino acid sequence of SEQ ID NO: 18, and CDR-H3 having the amino acid sequence of SEQ ID NO: 19; and a light chain variable region including CDR-L1 having the amino acid sequence of SEQ ID NO: 20, CDR-L2 having the amino acid sequence of SEQ ID NO: 21, and CDR-L3 having the amino acid sequence of SEQ ID NO: 22.

More specifically, in a specific embodiment of the present disclosure, the protein complex includes: an affibody specifically binding to the extracellular domain of CD137 and including an amino acid sequence represented by any one of SEQ ID NOs: 1 to 8; and
an antibody or antigen-binding fragment thereof including a heavy chain variable region including CDR-H1 having the amino acid sequence of SEQ ID NO: 17, CDR-H2 having the amino acid sequence of SEQ ID NO: 18, and CDR-H3 having the amino acid sequence of SEQ ID NO: 19; and a light chain variable region including CDR-L1 having the amino acid sequence of SEQ ID NO: 20, CDR-L2 having the amino acid sequence of SEQ ID NO: 21, and CDR-L3 having the amino acid sequence of SEQ ID NO: 22.

In a specific embodiment of the present disclosure, the component (b) includes: a heavy chain variable region including the amino acid sequence of SEQ ID NO: 23; and a light chain variable region including the amino acid sequence of SEQ ID NO: 24.

In another specific embodiment of the present disclosure, the protein complex includes: an affibody that specifically binds to the extracellular domain of CD137 according to an embodiment of the present disclosure; and

an antibody or antigen-binding fragment thereof including a heavy chain variable region including the amino acid sequence of SEQ ID NO: 23 and a light chain variable region including the amino acid sequence of SEQ ID NO: 24.

More specifically, in a specific embodiment of the present disclosure, the protein complex includes: an affibody that specifically binds to the extracellular domain of CD137 and includes an amino acid sequence represented by any one of SEQ ID NOs: 1 to 8; and
an antibody or antigen-binding fragment thereof including a heavy chain variable region including the amino acid sequence of SEQ ID NO: 23 and a light chain variable region including the amino acid sequence of SEQ ID NO: 24.

In an embodiment of the present disclosure, the antibody or antigen-binding fragment that specifically binds to HER2 includes:
(c) a heavy chain variable region including the amino acid sequence of SEQ ID NO: 28 and a light chain variable region including the amino acid sequence of SEQ ID NO: 29;
(d) trastuzumab; or
(e) a heavy chain variable region including CDR-H1 having the amino acid sequence of SEQ ID NO: 30, CDR-H2 having the amino acid sequence of SEQ ID NO: 31, and CDR-H3 having the amino acid sequence of SEQ ID NO: 32; and a light chain variable region including CDR-L1 having the amino acid sequence of SEQ ID NO: 33, CDR-L2 having the amino acid sequence of SEQ ID NO: 34, and CDR-L3 having the amino acid sequence of SEQ ID NO: 35.

Trastuzumab is a commercially available antibody that specifically binds to human HER2, and it will be understood by those skilled in the art that any antibody or antigen-binding fragment known to specifically bind to human HER2 may be used without limitation.

In a specific embodiment of the present disclosure, the protein complex includes: an affibody that specifically binds to the extracellular domain of CD137; and
(c) an antibody or antigen-binding fragment thereof including a heavy chain variable region including the amino acid sequence of SEQ ID NO: 28 and a light chain variable region including the amino acid sequence of SEQ ID NO: 29.

More specifically, in a specific embodiment of the present disclosure, the protein complex includes: an affibody that specifically binds to the extracellular domain of CD137 and includes an amino acid sequence represented by any one of SEQ ID NOs: 1 to 8; and
(c) an antibody or antigen-binding fragment including a heavy chain variable region including the amino acid sequence of SEQ ID NO: 28 and a light chain variable region including the amino acid sequence of SEQ ID NO: 29.

In a specific embodiment of the present disclosure, the protein complex includes: an affibody that specifically binds to the extracellular domain of CD137; and
(d) trastuzumab.

More specifically, in a specific embodiment of the present disclosure, the protein complex includes: an affibody that specifically binds to the extracellular domain of CD137 and includes an amino acid sequence represented by any one of SEQ ID NOs: 1 to 8; and
(d) trastuzumab.

In a specific embodiment of the present disclosure, the protein complex includes: an affibody that specifically binds to the extracellular domain of CD137; and
(e) an antibody or antigen-binding fragment including a heavy chain variable region including CDR-H1 having the amino acid sequence of SEQ ID NO: 30, CDR-H2 having the amino acid sequence of SEQ ID NO: 31, and CDR-H3 having the amino acid sequence of SEQ ID NO: 32; and a light chain variable region including CDR-L1 having the amino acid sequence of SEQ ID NO: 33, CDR-L2 having the amino acid sequence of SEQ ID NO: 34, and CDR-L3 having the amino acid sequence of SEQ ID NO: 35.

More specifically, in a specific embodiment of the present disclosure, the protein complex includes: an affibody that specifically binds to the extracellular domain of CD137 and includes an amino acid sequence represented by any one of SEQ ID NOS: 1 to 8; and
(e) an antibody or antigen-binding fragment comprising a heavy chain variable region including CDR-H1 having the amino acid sequence of SEQ ID NO: 30, CDR-H2 having the amino acid sequence of SEQ ID NO: 31, and CDR-H3 having the amino acid sequence of SEQ ID NO: 32; and a light chain variable region including CDR-L1 having the amino acid sequence of SEQ ID NO: 33, CDR-L2 having the amino acid sequence of SEQ ID NO: 34, and CDR-L3 having the amino acid sequence of SEQ ID NO: 35.

In a specific embodiment of the present disclosure, the component (e) includes a heavy chain variable region including the amino acid sequence of SEQ ID NO: 36 and a light chain variable region including the amino acid sequence of SEQ ID NO: 37.

More specifically, in a specific embodiment of the present disclosure, the protein complex includes: an affibody that specifically binds to the extracellular domain of CD137 and includes an amino acid sequence represented by any one of SEQ ID NOS: 1 to 8; and
an antibody or antigen-binding fragment including a heavy chain variable region including the amino acid sequence of SEQ ID NO: 36 and a light chain variable region including the amino acid sequence of SEQ ID NO: 37.

In an embodiment of the present disclosure, the antibody or antigen-binding fragment that specifically binds to CD19 includes:
(f) a heavy chain variable region including the amino acid sequence of SEQ ID NO: 38 and a light chain variable region comprising the amino acid sequence of SEQ ID NO: 39; or
(g) FMC63.

FMC63 is a commercially available antibody that specifically binds to human CD19, and it will be understood by those skilled in the art that any antibody or antigen-binding fragment known to specifically bind to human CD19 may be used without limitation.

In a specific embodiment of the present disclosure, the protein complex includes: an affibody that specifically binds to the extracellular domain of CD137; and
(f) an antibody or antigen-binding fragment including a heavy chain variable region including the amino acid sequence of SEQ ID NO: 38 and a light chain variable region including the amino acid sequence of SEQ ID NO: 39.

More specifically, in a specific embodiment of the present disclosure, the protein complex includes: an affibody that specifically binds to the extracellular domain of CD137 and includes an amino acid sequence represented by any one of SEQ ID NOs: 1 to 8; and
(f) an antibody or antigen-binding fragment including a heavy chain variable region including the amino acid sequence of SEQ ID NO: 38 and a light chain variable region including the amino acid sequence of SEQ ID NO: 39.

In a specific embodiment of the present disclosure, the protein complex include: an affibody that specifically binds to the extracellular domain of CD137; and
(g) FMC63.

More specifically, in a specific embodiment of the present disclosure, the protein complex includes: an affibody that specifically binds to the extracellular domain of CD137 and includes an amino acid sequence represented by any one of SEQ ID NOs: 1 to 8; and
(g) FMC63.

Another aspect of the present disclosure provides a nucleic acid molecule including a nucleotide sequence encoding a protein complex according to one embodiment of the present disclosure.

In one embodiment of the present disclosure, the nucleic acid molecule includes a nucleotide sequence coding for a protein complex including an affibody that specifically binds to the extracellular domain of CD137 and an antibody or antigen-binding fragment thereof that specifically binds to a protein present on the surface of a cancer cell.

In a specific embodiment of the present disclosure, the nucleic acid molecule includes a nucleotide sequence coding for a protein complex including an affibody that specifically binds to the extracellular domain of CD137 and an antibody or antigen-binding fragment thereof that specifically binds to EGFR.

In another specific embodiment of the present disclosure, the nucleic acid molecule includes a nucleotide sequence coding for a protein complex including an affibody that specifically binds to the extracellular domain of CD137 and an antibody or antigen-binding fragment thereof that specifically binds to HER2.

In yet another specific embodiment of the present disclosure, the nucleic acid molecule includes a nucleotide sequence coding for a protein complex including an affibody that specifically binds to the extracellular domain of CD137 and an antibody or antigen-binding fragment thereof that specifically binds to CD19.

The nucleotide sequence encoding the protein complex of the present disclosure may be any nucleotide sequence that encodes the amino acid sequence constituting the protein complex, and is not limited to any particular nucleotide sequence, as will be apparent to those skilled in the art.

Provided according to another aspect of the present disclosure is a recombinant vector carrying a nucleic acid molecule coding for the above-described protein complex.

According to an embodiment of the present disclosure, in the vector of the present disclosure, the nucleic acid molecules coding for: an affibody that specifically binds to the extracellular domain of CD137 and an antibody or antigen-binding fragment thereof that specifically binds to a protein on the surface of a cancer cell are operatively linked to a promoter.

According to a specific embodiment of the present disclosure, in the vector of the present disclosure, the nucleic acid molecules coding for: an affibody that specifically binds to the extracellular domain of CD137; and an antibody or antigen-binding fragment thereof that specifically binds to EGFR, HER2, or CD19 are operatively linked to a promoter.

According to a specific embodiment of the present disclosure, in the vector of the present disclosure, the nucleic acid molecules coding for: the heavy chain variable region and the light chain variable region of the antibody are operatively linked to a promoter.

According to another specific embodiment of the present disclosure, in the vector of the present disclosure, the nucleic acid molecule coding for: an affibody that specifically binds to the extracellular domain of CD137; and cetuximab are operatively linked to a promoter.

According to another specific embodiment of the present disclosure, in the vector of the present disclosure, the nucleic acid molecules coding for: an affibody that specifically binds to the extracellular domain of CD137; and
a heavy chain variable region including CDR-H1 of SEQ ID NO: 17, CDR-H2 of SEQ ID NO: 18, and CDR-H3 of SEQ ID NO: 19; and a light chain variable region including CDR-L1 of SEQ ID NO: 20, CDR-L2 of SEQ ID NO: 21, and CDR-L3 of SEQ ID NO: 22 are operatively linked to a promoter.

According to yet another specific embodiment of the present disclosure, in the vector of the present disclosure, the nucleic acid molecules coding for: an affibody that specifically binds to the extracellular domain of CD137; and
a heavy chain variable region including the amino acid sequence of SEQ ID NO: 23 and a light chain variable region including the amino acid sequence of SEQ ID NO: 24 are operatively linked to a promoter.

According to a specific embodiment of the present disclosure, in the vector of the present disclosure, the nucleic acid molecules coding for: an affibody that specifically binds to the extracellular domain of CD137; and
a monoclonal antibody or antigen-binding fragment thereof that specifically binds to HER2 are operatively linked to a promoter.

According to another specific embodiment of the present disclosure, in the vector of the present disclosure, the nucleic acid molecules coding for: an affibody that specifically binds to the extracellular domain of CD137; and
(c) a heavy chain variable region comprising the amino acid sequence of SEQ ID NO: 28 and a light chain variable region comprising the amino acid sequence of SEQ ID NO: 29;
(d) trastuzumab, or
(e) a heavy chain variable region comprising CDR-H1 of SEQ ID NO: 30, CDR-H2 of SEQ ID NO: 31, and CDR-H3 of SEQ ID NO: 32; and a light chain variable region comprising CDR-L1 of SEQ ID NO: 33, CDR-L2 of SEQ ID NO: 34, and CDR-L3 of SEQ ID NO: 35 are operatively linked to a promoter.

According to yet another specific embodiment of the present disclosure, in the vector of the present disclosure, the nucleic acid molecules coding for: an affibody that specifically binds to the extracellular domain of CD137; and
a heavy chain variable region comprising the amino acid sequence of SEQ ID NO: 36 and a light chain variable region comprising the amino acid sequence of SEQ ID NO: 37 are operatively linked to a promoter.

According to another specific embodiment of the present disclosure, in the vector of the present disclosure, the nucleic acid molecules coding for: an affibody that specifically binds to the extracellular domain of CD137; and
a monoclonal antibody or antigen-binding fragment thereof that specifically binds to CD19 are operatively linked to a promoter.

According to another specific embodiment of the present disclosure, in the vector of the present disclosure, the nucleic acid molecules coding for: an affibody that specifically binds to the extracellular domain of CD137; and
(f) a heavy chain variable region comprising the amino acid sequence of SEQ ID NO: 38 and a light chain variable region comprising the amino acid sequence of SEQ ID NO: 39; or
(g) FMC63 are operatively linked to a promoter.

As used herein, the term "operatively linked" refers to a functional connection between a nucleic acid expression control sequence (e.g., a promoter, signal sequence, or an array of transcription factor binding sites) and another nucleic acid sequence, such that the control sequence regulates the transcription and/or translation of the other nucleic acid sequence.

The recombinant vector system of the present disclosure may be constructed using various methods well known in the art, such as those described in Sambrook et al., Molecular Cloning: A Laboratory Manual, Cold Spring Harbor Laboratory Press (2001), which is incorporated herein by reference.

The vector of the present disclosure may be constructed as a cloning vector or as an expression vector. In addition, the vector may be constructed using either a prokaryotic or eukaryotic host.

For example, when the vector of the present disclosure is an expression vector and the host is a eukaryotic cell, promoters derived from mammalian genomes (e.g., metallothionein promoter, beta-actin promoter, human hemoglobin promoter, and human muscle creatine promoter) or from mammalian viruses (e.g., adenovirus late promoter, vaccinia virus 7.5K promoter, SV40 promoter, cytomegalovirus promoter, HSV tk promoter, mouse mammary tumor virus (MMTV) promoter, HIV LTR promoter, Moloney virus promoter, Epstein-Barr virus (EBV) promoter, and Rous sarcoma virus (RSV) promoter) may be used, typically in combination with a polyadenylation sequence as a transcription termination signal.

The vector of the present disclosure may be fused to other sequences to facilitate purification of the antibody expressed therefrom. Examples of fusion partners include glutathione S-transferase (Pharmacia, USA), maltose-binding protein (NEB, USA), FLAG (IBI, USA), and 6xHis (hexahistidine; Qiagen, USA).

Furthermore, since the protein expressed by the vector of the present disclosure is an antibody, the expressed antibody can be easily purified without additional fusion sequences, for example, using a protein A column.

The expression vector of the present disclosure may include, as a selection marker, an antibiotic resistance gene commonly used in the art, such as resistance to ampicillin, gentamicin, carbenicillin, chloramphenicol, streptomycin, kanamycin, geneticin, neomycin, or tetracycline.

Optionally, the vector may further carry a gene encoding a reporter molecule, such as luciferase or β-glucuronidase.

According to an embodiment of the present disclosure, the expression vector is a host cell expression vector carrying a nucleic acid molecule coding for an antibody or antigen-binding fragment thereof specific to EGFR, HER2, or CD19, a promoter operatively linked to the nucleotide sequence of the nucleic acid molecule and functioning to form an RNA molecule in a host cell, and a poly A signal sequence that functions in the host cell to cause polyadenylation at the 3'-end of the RNA molecule.

Provided according to an aspect of the present disclosure is an isolated host cell including the above-described recombinant vector.

In an embodiment of the present disclosure, the isolated host cell is a cell transformed with the recombinant vector of the present disclosure.

Host cells capable of stably and continuously cloning and expressing the vector of the present disclosure are well known in the art, and any such host cell may be used. Suitable eukaryotic host cells for the vector include, but are not limited to, *Saccharomyces cerevisiae* (yeast), insect cells, monkey kidney cells (e.g., COS7), NSO cells, SP2/0 cells, Chinese hamster ovary (CHO) cells, W138 cells, baby hamster kidney (BHK) cells, MDCK cells, myeloma cell lines, HuT 78 cells, and HEK-293 cells.

In an embodiment of the present disclosure, the host cell may be selected from the group consisting of cynomolgus monkey-derived fibroblasts (CYNOM-K1, Sigma-Aldrich), rhesus monkey-derived kidney cells (ATCC, LLC-MK2 Original CCL-7^{™} or ATCC, FRhK-4, .CRL-1688^{™}), and rhesus monkey-derived lymphoblasts (ATCC, LCL 8664, CRL-1805^{™}).

Another aspect of the present disclosure provides a pharmaceutical composition for preventing or treating cancer, the composition including the protein complex according to an embodiment of the present disclosure and a pharmaceutically acceptable carrier.

Since the pharmaceutical composition of the present disclosure uses the aforementioned protein complex as an active ingredient, the overlapping descriptions are omitted herein to avoid undue complexity.

In an embodiment of the present disclosure, the cancer may be a hematologic cancer or a solid tumor.

In a specific embodiment of the present disclosure, the solid tumor may be selected from the group consisting of brain tumor, benign astrocytoma, malignant astrocytoma, pituitary adenoma, meningioma, brain lymphoma, oligodendroglioma, ependymoma, brainstem tumor, head and neck cancer, laryngeal cancer, oropharyngeal cancer, nasal cancer, sinus cancer, nasosinus cancer, nasopharyngeal cancer, salivary gland cancer, hypopharyngeal cancer, thyroid cancer, oral cancer, thoracic tumor, small cell lung cancer, non-small cell lung cancer, thymic cancer, mediastinal tumor, esophageal cancer, breast cancer, male breast cancer, abdominal tumor, gastric cancer, liver cancer, gallbladder cancer, bile duct cancer, pancreatic cancer, small intestine cancer, colon cancer, rectal cancer, anal cancer, bladder cancer, kidney cancer, male genital cancers, penile cancer, prostate cancer, female genital cancers, cervical cancer, endometrial cancer, ovarian cancer, uterine sarcoma, vaginal cancer, vulvar cancer, female urethral cancer, and skin cancer, but with no limitations thereto.

In a specific embodiment of the present disclosure, the hematologic cancer may be selected from the group consisting of acute myeloid leukemia, chronic myeloid leukemia, myelodysplastic syndrome, myeloproliferative neoplasms, polycythemia vera, essential thrombocythemia, myelofibrosis, monocytic leukemia, erythroleukemia, megaloblastic leukemia, basophilic leukemia, eosinophilic leukemia, acute lymphoblastic leukemia, chronic lymphocytic leukemia, multiple myeloma, lymphoma, B-cell lymphoma, marginal zone B-cell lymphoma, Hodgkin's lymphoma, non-Hodgkin's lymphoma, diffuse large B-cell lymphoma, extranodal marginal zone lymphoma, extranodal NK/T-cell lymphoma, peripheral T-cell lymphoma, Burkitt lymphoma, precursor cell neoplasms, mantle cell lymphoma, follicular lymphoma, immunodeficiency-associated lymphoproliferative disorders, nodular lymphocyte predominant Hodgkin lymphoma, nodular sclerosis classical Hodgkin lymphoma, lymphocyte-rich classical Hodgkin lymphoma, mixed cellularity classical Hodgkin lymphoma, lymphocyte-depleted classical Hodgkin lymphoma, and chemotherapy-resistant blastoid leukemia, but with no limitations thereto.

In an embodiment of the present disclosure, the protein complex induces T-cell activation.

Specifically, since the protein complex of the present disclosure specifically binds to a protein on the surface of a cancer cell and simultaneously to CD137 on a T cell, the complex acts as a T-cell agonist, thereby inducing T-cell activation and reducing or inhibiting the growth of cancer cells. Accordingly, it may be effectively used in the prevention or treatment of cancer. That is, the protein complex of the present disclosure induces T-cell activation in a tumor antigen-dependent manner.

More specifically, the protein complex of the present disclosure specifically binds to EGFR on the surface of a cancer cell and simultaneously to CD137 on a T cell, and thus acts as a T-cell agonist, inducing T-cell activation, thereby reducing the number of EGFR-expressing cancer cells or inhibiting their proliferation. Therefore, it is useful for the prevention or treatment of cancer. In other words, the protein complex induces T-cell activation in an EGFR-dependent manner.

The cancer may be EGFR-positive.

The cancer may be characterized by EGFR overexpression.

The cancer may be, but is not limited to, non-small cell lung cancer (NSCLC), lung cancer, colorectal cancer, brain tumor, astrocytoma, esophageal cancer, cervical cancer, or synovial sarcoma.

The cancer may be triple-negative breast cancer (TNBC). TNBC is known to overexpress EGFR. TNBC refers to breast cancer in which the expression of estrogen receptor (ER), progesterone receptor (PR), and human epidermal growth factor receptor 2 (Her2neu) is less than 1%.

More specifically, the protein complex of the present disclosure specifically binds to HER2 on the surface of a cancer cell and simultaneously to CD137 on a T cell, and thus acts as a T-cell agonist, inducing T-cell activation, thereby reducing the number of HER2-expressing cancer cells or inhibiting their proliferation. Therefore, it is useful for the prevention or treatment of cancer. That is, the bispecific antibody of the present disclosure induces T-cell activation in a HER2-dependent manner.

The cancer may be HER2-positive.

The cancer may be characterized by HER2 overexpression.

The cancer may be invasive breast cancer or metastatic breast cancer. It may also be breast cancer, bladder cancer, pancreatic cancer, ovarian cancer, or gastric cancer, but is not limited thereto.

More specifically, the protein complex of the present disclosure specifically binds to CD19 on the surface of a cancer cell and simultaneously to CD137 on a T cell, and thus acts as a T-cell agonist, inducing T-cell activation, thereby reducing the number of CD19-expressing cancer cells or inhibiting their proliferation. Therefore, it is useful for the prevention or treatment of cancer. That is, the protein complex of the present disclosure induces T-cell activation in a CD19-dependent manner.

The cancer may be CD19-positive.

The cancer may be characterized by CD19 overexpression.

The cancer may be lymphoma. The lymphoma may be B-cell lymphoma. The cancer may be marginal zone lymphoma, Hodgkin's lymphoma, non-Hodgkin's lymphoma, chronic lymphocytic leukemia (CLL), acute lymphoblastic leukemia (ALL), myeloid leukemia, or chemotherapy-resistant blastoid leukemia, but is not limited thereto.

In an embodiment of the present disclosure, the activation of T cells results in increased production of interferon-gamma (IFN-y).

In another embodiment of the present disclosure, the pharmaceutical composition increases interferon-gamma in a cancer cell surface protein-dependent manner.

In a specific embodiment of the present disclosure, the pharmaceutical composition increases interferon-gamma in an EGFR-dependent manner. That is, when the composition comes into contact with EGFR-positive or EGFR-overexpressing cancer cells, it activates T cells and increases the concentration of interferon-gamma.

In another specific embodiment of the present disclosure, the pharmaceutical composition increases interferon-gamma in a HER2-dependent manner. That is, when the composition contacts HER2-positive or HER2-overexpressing cancer cells, it activates T cells and increases the concentration of interferon-gamma.

In another specific embodiment of the present disclosure, the pharmaceutical composition increases interferon-gamma in a CD19-dependent manner. That is, when the composition contacts CD19-positive or CD19-overexpressing cancer cells, it activates T cells and increases the concentration of interferon-gamma.

In another embodiment of the present disclosure, the pharmaceutical composition induces T-cell activation in a cancer cell surface protein-dependent manner.

In a specific embodiment of the present disclosure, the pharmaceutical composition induces T-cell activation in an EGFR-dependent manner. That is, when the composition contacts EGFR-positive or EGFR-overexpressing cancer cells, it induces T-cell activation.

In another specific embodiment of the present disclosure, the pharmaceutical composition induces T-cell activation in a HER2-dependent manner. That is, when the composition contacts HER2-positive or HER2-overexpressing cancer cells, it induces T-cell activation.

In another specific embodiment of the present disclosure, the pharmaceutical composition induces T-cell activation in a CD19-dependent manner. That is, when the composition contacts CD19-positive or CD19-overexpressing cancer cells, it induces T-cell activation.

The pharmaceutically acceptable carrier included in the pharmaceutical composition of the present disclosure is one that is commonly used in formulation, and may include, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylparaben, propylparaben, talc, magnesium stearate, and mineral oil. The pharmaceutical composition of the present disclosure may further include lubricants, humectants, sweeteners, flavoring agents, emulsifiers, suspending agents, preservatives, and the like. For details of suitable pharmaceutically acceptable carriers and preparations, reference may be made to Remington's Pharmaceutical Sciences (19th ed., 1995).

The pharmaceutical composition of the present disclosure may be administered orally or parenterally, for example, via intravenous injection, subcutaneous injection, intramuscular injection, intraperitoneal injection, intra-sternal injection, topical administration, intranasal administration, pulmonary administration, or rectal administration.

The appropriate dosage of the pharmaceutical composition of the present disclosure may vary depending on formulation method, mode of administration, patient's age, body weight, sex, pathological condition, diet, time of administration, route of administration, excretion rate, and responsiveness. A skilled physician can readily determine and prescribe a dosage that is effective for the desired treatment or prevention. In a preferred embodiment of the present disclosure, the daily dosage of the pharmaceutical composition is from 0.0001 to 100 mg/kg. As used herein, the term "pharmaceutically effective amount" refers to an amount sufficient to prevent or treat the aforementioned disease.

As used herein, the term "prevention" refers to prophylactic treatment or protection against a disease or disease condition. The term "treatment" refers to alleviation, suppression, mitigation, or eradication of a disease condition.

The pharmaceutical composition of the present disclosure may be formulated into a unit dosage form or placed into a multi-dose container by a method easily implementable by those skilled in the art, using a pharmaceutically acceptable carrier and/or excipient. The formulation may be in the form of a solution, suspension, or emulsion in an oil- or water-based medium; or in the form of extracts, powders, suppositories, granules, tablets, or capsules. It may additionally contain a dispersant or stabilizer.

### Advantageous Effects of Invention

The features and advantages of the present disclosure may be summarized as follows:
The present disclosure provides an affibody that specifically binds to an extracellular domain of CD137.

The present disclosure provides a protein complex including: an affibody that specifically binds to an extracellular domain of CD137; and an antibody or an antigen-binding fragment thereof that specifically binds to a protein on the surface of a cancer cell.

The present disclosure provides a pharmaceutical composition for preventing or treating cancer, the composition including the aforementioned affibody or protein complex and a pharmaceutically acceptable carrier.

By using the affibody that specifically binds to CD137, the present disclosure enables the development of a protein complex that not only specifically binds to T cells but also to antigens on the surface of various cancer cells, and thus can be advantageously used for the prevention or treatment of both hematologic cancers and solid tumors.

### Brief Description of Drawings

FIG. 1 shows the results of detecting an affibody that binds to CD137 in the form of a periplasmic extract.
FIG. 2 shows the results of confirming an affibody that binds to CD137 expressed on cells, using CD137-positive activated CEMT cells in the form of an antibody-affibody construct.
FIGS. 3a to 3d show the results of confirming whether bispecific antibodies, produced using two EGFR-binding antibodies (CET or 15E3) and CD137-binding affibodies, bind to both CD137 and EGFR proteins.

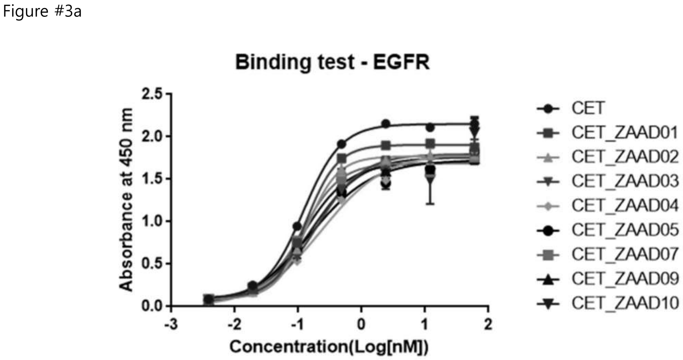

FIG. 3a shows that the bispecific antibody constructed with a CD137-binding affibody and cetuximab (CET) binds to EGFR protein.

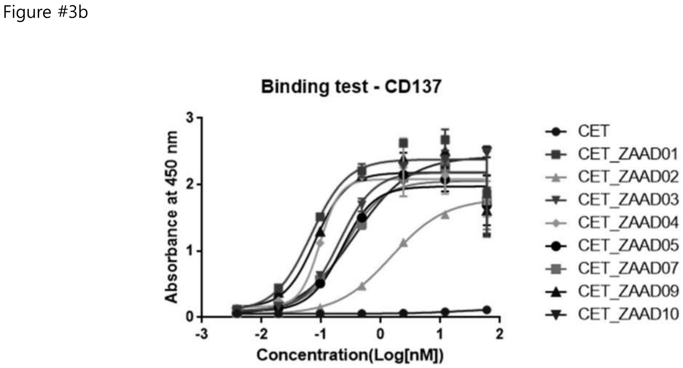

FIG. 3b shows that the bispecific antibody constructed with a CD137-binding affibody and cetuximab (CET) binds to CD137 protein.

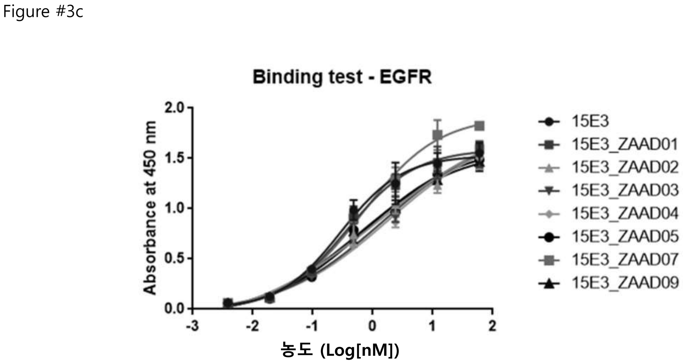

FIG. 3c shows that the bispecific antibody constructed with a CD137-binding affibody and 15E3 antibody binds to EGFR protein.

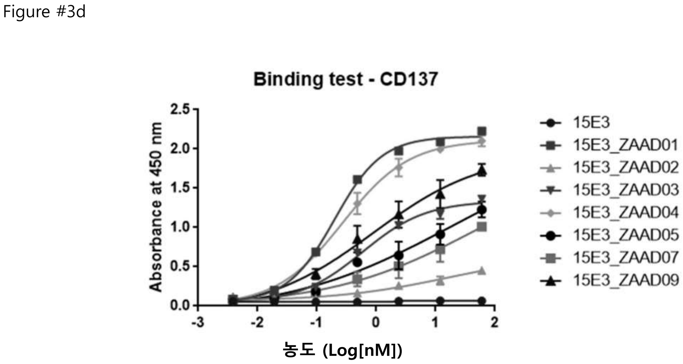

FIG. 3d shows that the bispecific antibody constructed with a CD137-binding affibody and 15E3 antibody binds to CD137 protein.

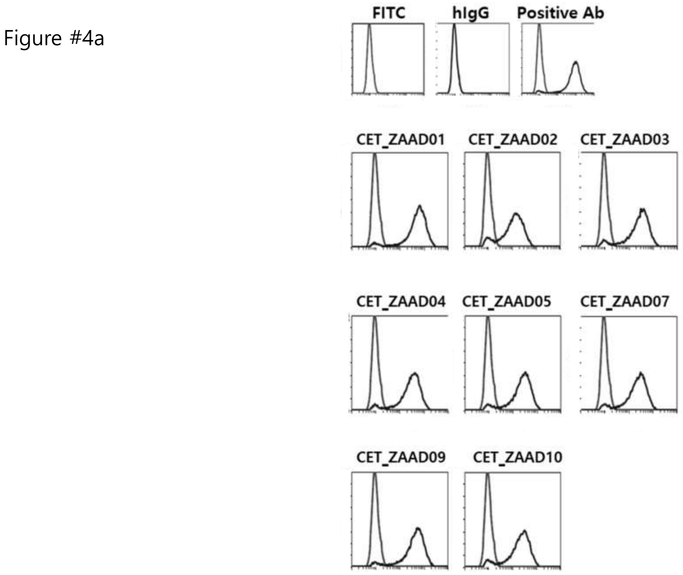

FIG. 4a shows that the bispecific antibody constructed with a CD137-binding affibody and cetuximab (CET) binds to CD137-expressing activated CEMT cells.

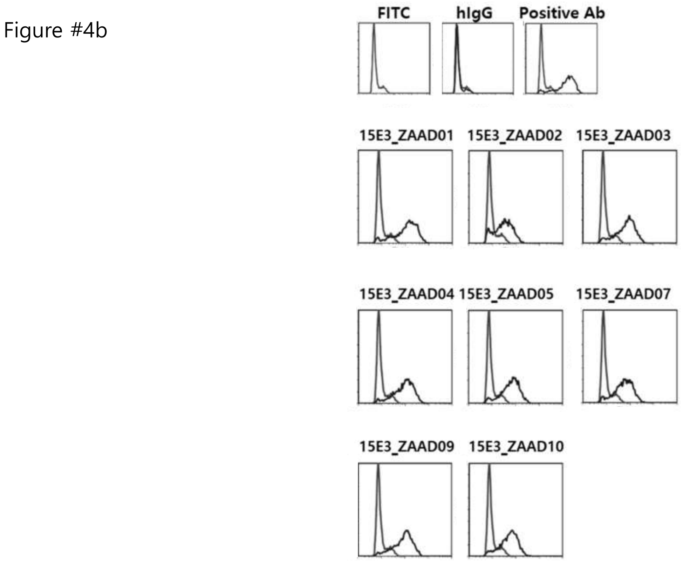

FIG. 4b shows that the bispecific antibody constructed with a CD137-binding affibody and 15E3 antibody binds to CD137-expressing activated CEMT cells.

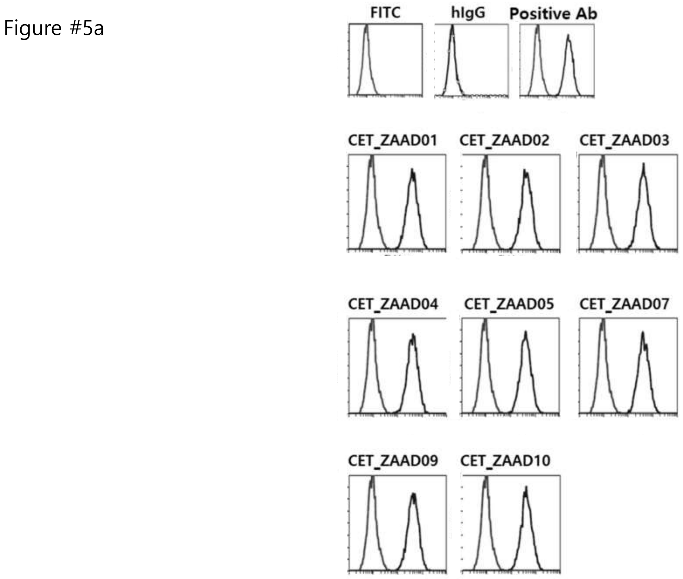

FIG. 5a shows that the bispecific antibody constructed with a CD137-binding affibody and cetuximab (CET) binds to EGFR-expressing HT29 cells.

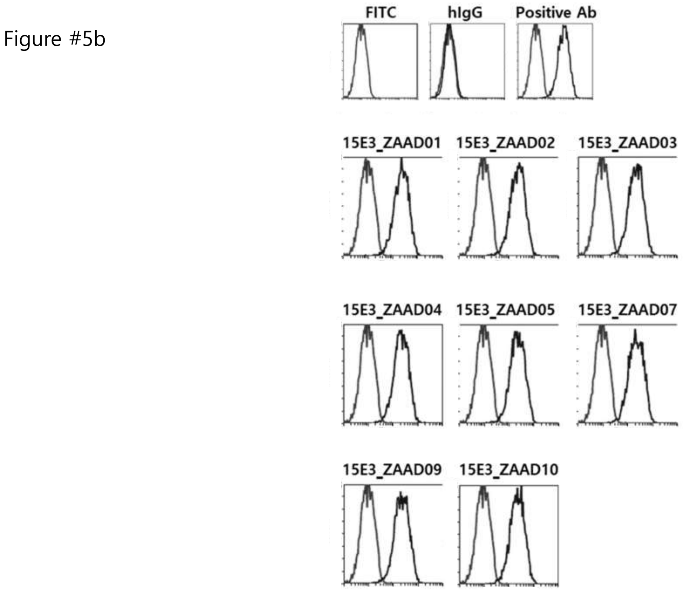

FIG. 5b shows that the bispecific antibody constructed with a CD137-binding affibody and 15E3 antibody binds to EGFR-expressing HT29 cells.

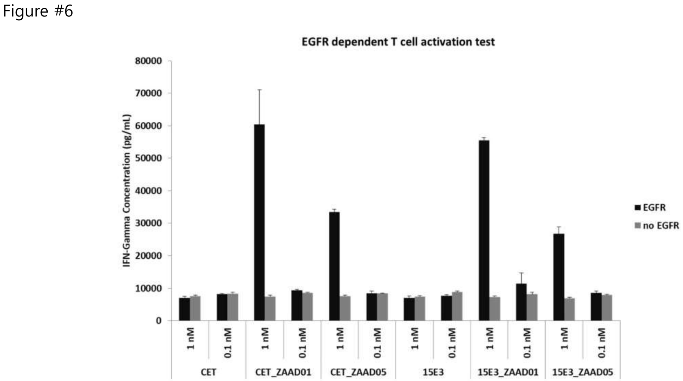

FIG. 6 shows the result of EGFR-dependent T cell activation confirmed by IFN-gamma secretion analysis to screen CD137-binding affibodies.

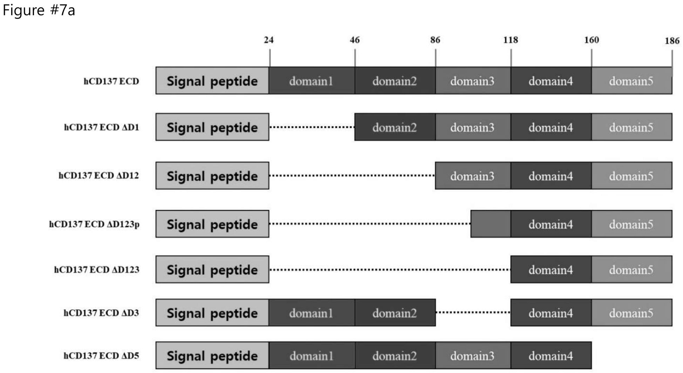

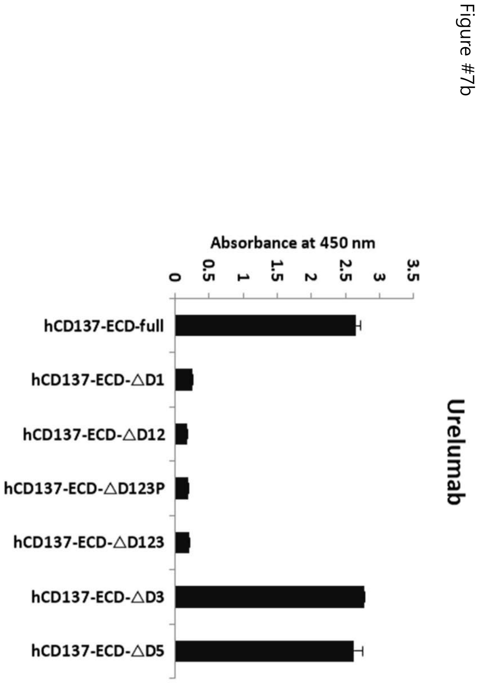

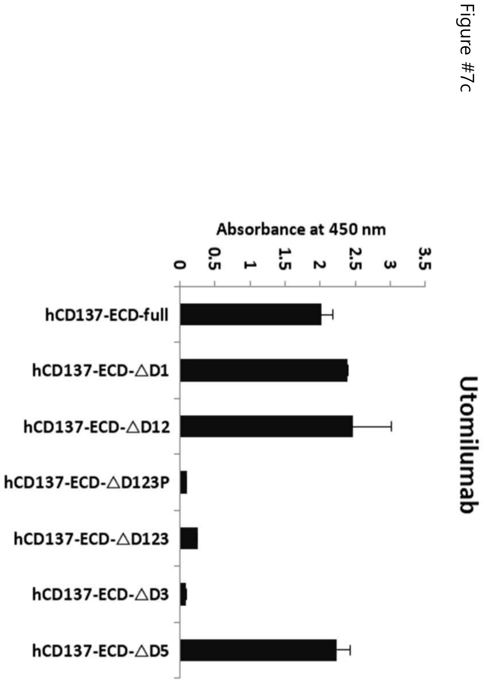

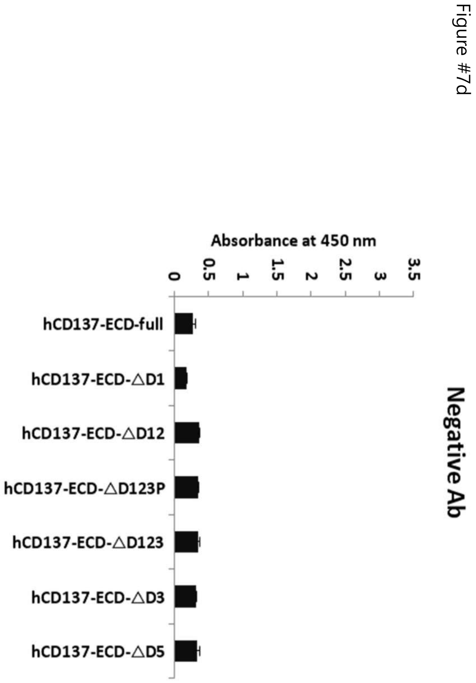

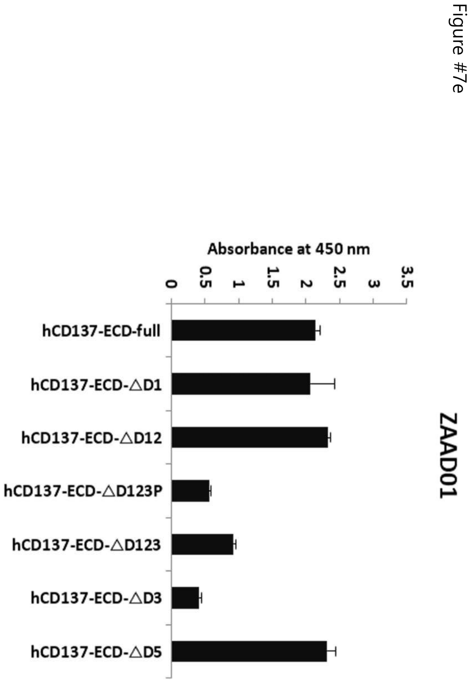

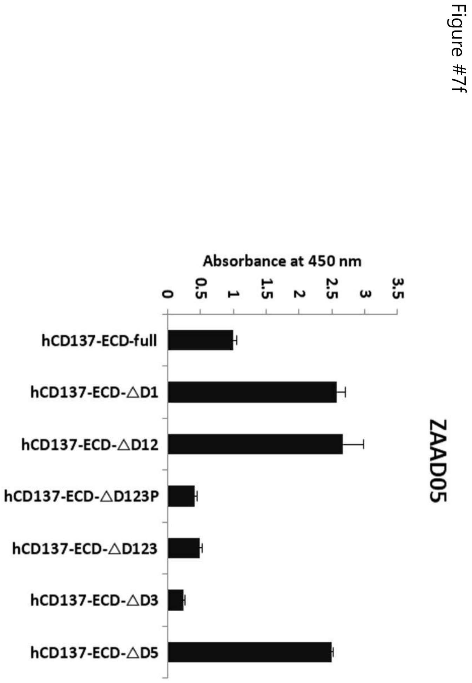

FIG. 7 shows the binding domains of two selected CD137-binding affibodies on the extracellular domain (ECD) of CD137.
FIG. 7a shows a schematic of hCD137-ECD, hCD137-ECD-ΔD1, hCD137-ECD-ΔD12, hCD137-ECD-ΔD123p, hCD137-ECD-ΔD123, hCD137-ECD-ΔD3, and hCD137-ECD-ΔD5, each comprising combinations of a signal peptide and different domains 1 to 5 of the human CD137 ECD. The amino acid sequence of the CD137 ECD consisting of domains 1 to 5 is shown in Sequence ID No. 27.
FIG. 7b shows that urelumab binds to domain 1 of the CD137 ECD.
FIG. 7c shows that utomilumab binds to domain 3 of the CD137 ECD.
FIG. 7d shows that cetuximab (negative Ab) does not bind to any domain of the CD137 ECD.
FIG. 7e shows that affibody ZAAD01 binds to domain 3 of the CD137 ECD.
FIG. 7f shows that affibody ZAAD05 binds to domain 3 of the CD137 ECD.

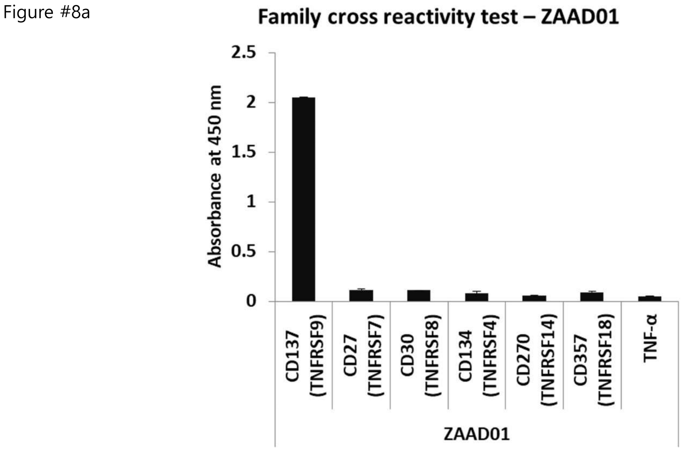

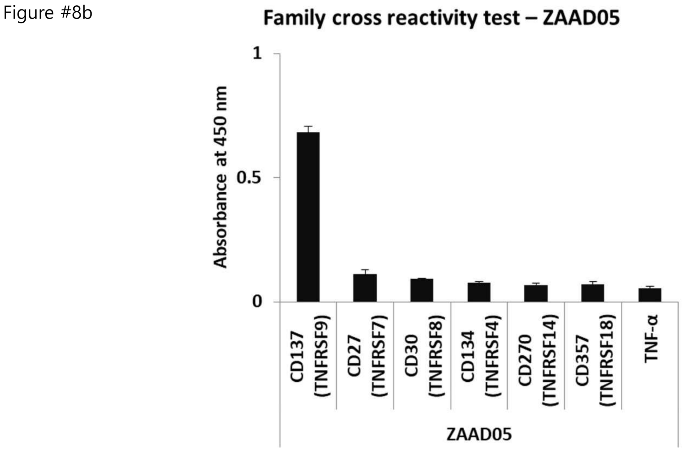

FIG. 8 shows the cross-reactivity of the two selected CD137-binding affibodies with other TNFR superfamily members: CD27, CD30, CD134, CD270, CD357, and TNF-α.
FIG. 8a shows that affibody ZAAD01 specifically binds only to CD137.
FIG. 8b shows that affibody ZAAD05 specifically binds only to CD137.

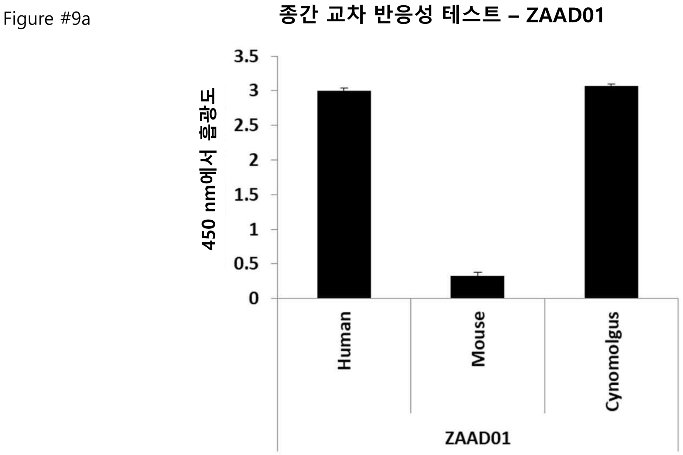

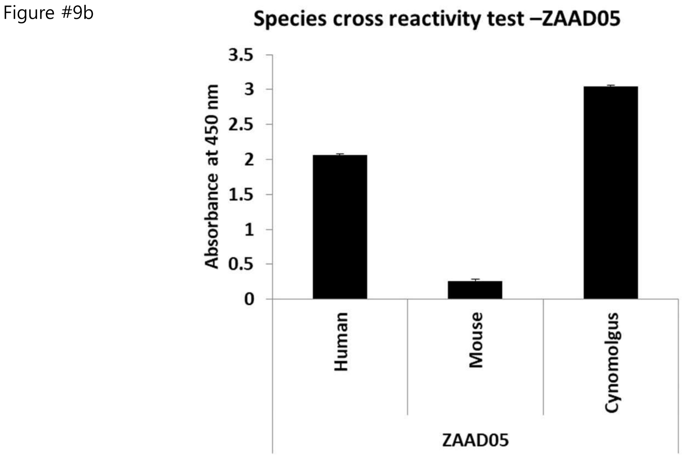

FIG. 9 shows interspecies cross-reactivity of the two selected CD137-binding affibodies with human, mouse, and cynomolgus monkey CD137 proteins.
FIG. 9a shows that affibody ZAAD01 binds to CD137 proteins of both human and cynomolgus monkey.
FIG. 9b shows that affibody ZAAD05 binds to CD137 proteins of both human and cynomolgus monkey.

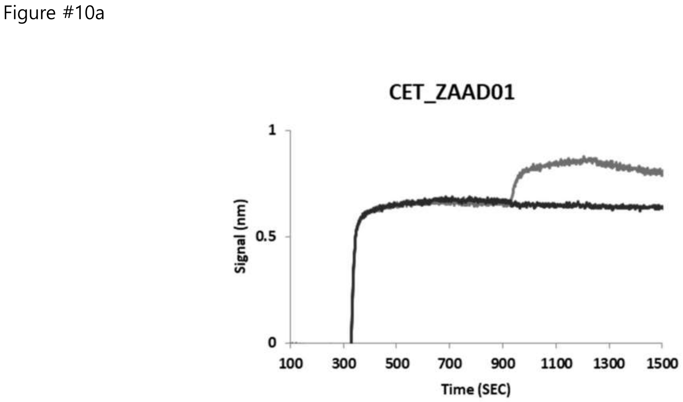

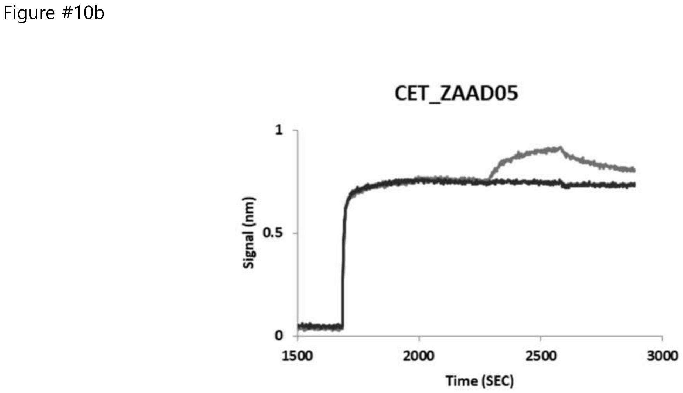

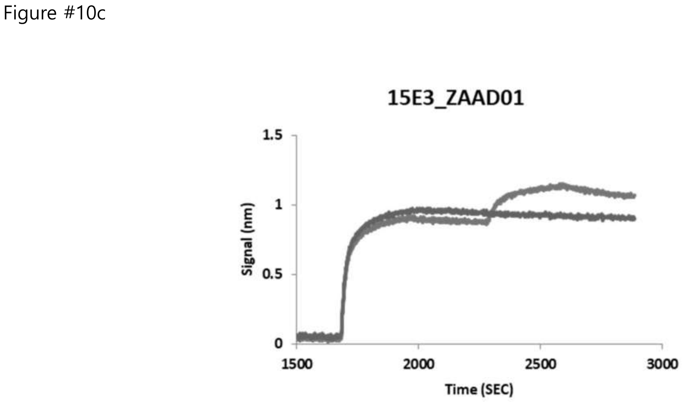

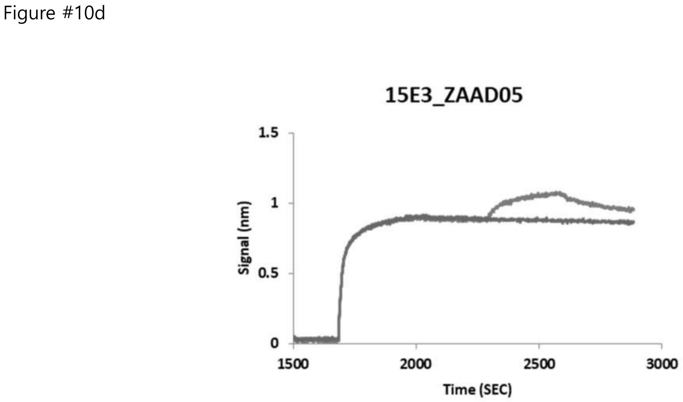

FIG. 10 shows binding of four antibody-affibody bispecific antibodies to both CD137 and EGFR using BioLayer Interferometry (BLI). FIG. 10a: CET-ZAAD01, FIG. 10b: CET-ZAAD05, FIG. 10c: 15E3-ZAAD01, FIG. 10d: 15E3-ZAAD05.

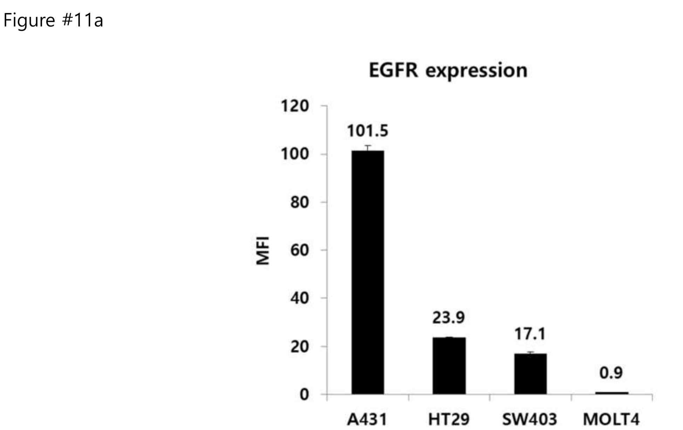

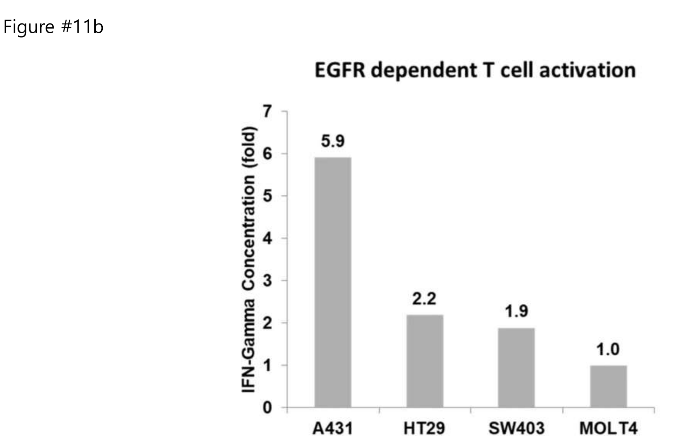

FIG. 11 shows correlation between EGFR expression levels on various cells and the degree of T cell activation by the bispecific antibodies, determined by flow cytometry and IFN-gamma secretion.
FIG. 11a shows EGFR expression in EGFR-positive cells (A431, HT29, and SW403) and EGFR-negative cells (MOLT4).
FIG. 11b shows that 15E3-ZAAD01 bispecific antibody induces EGFR-dependent T cell activation proportional to EGFR expression, based on IFN-gamma secretion.

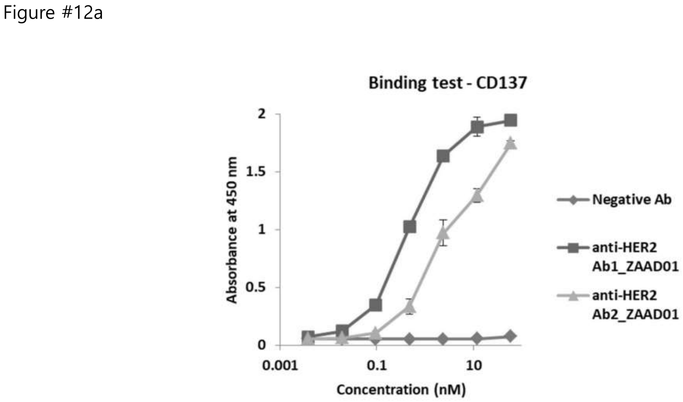

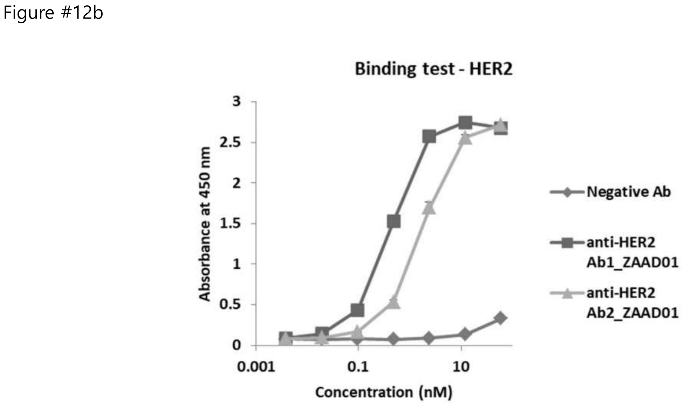

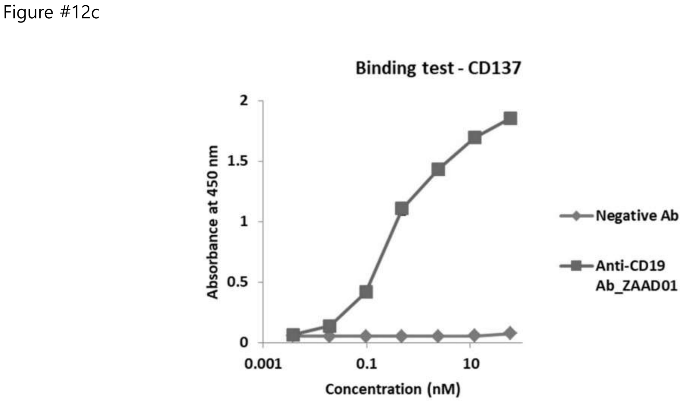

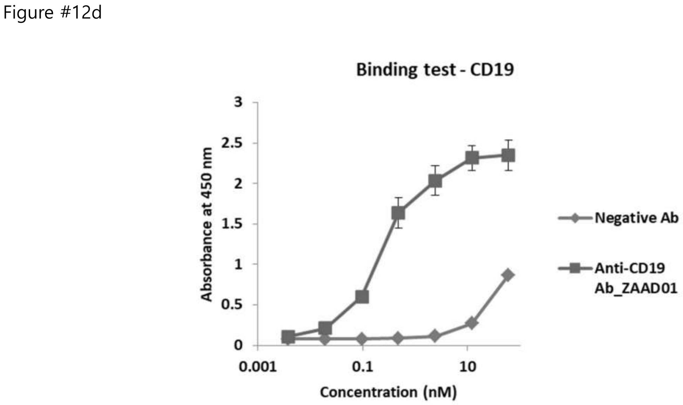

FIG. 12 shows that bispecific antibodies produced using CD137 affibody and HER2 or CD19 antibodies bind to both CD137 and HER2 or CD19, respectively.
FIG. 12a shows that trastuzumab-ZAAD01 (anti-HER2 Ab1_ZAAD01) and hz1E11.10-ZAAD01 (anti-HER2 Ab2_ZAAD01) bind to CD137.
FIG. 12b shows that trastuzumab-ZAAD01 and hz1E11.10-ZAAD01 bind to HER2.
FIG. 12c shows that FMC63-ZAAD01 (anti-CD19 Ab_ZAAD01) binds to CD137.
FIG. 12d shows that FMC63-ZAAD01 binds to CD19.

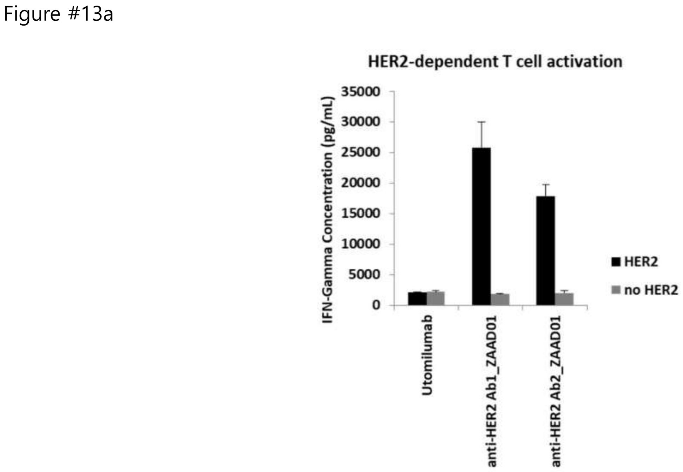

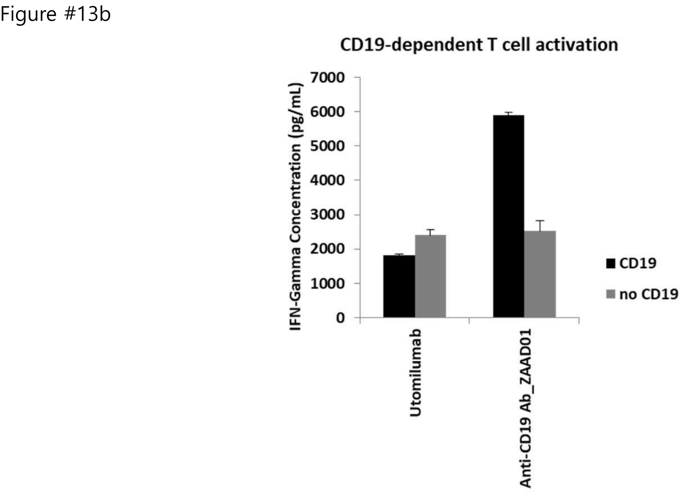

FIG. 13 shows that the bispecific antibodies constructed using CD137 affibody and HER2 or CD19 antibodies induce T cell activation in a HER2- or CD19-dependent manner, as measured by IFN-gamma secretion.
FIG. 13a shows that trastuzumab-ZAAD01 and hz1E11.10-ZAAD01 activate T cells in a HER2-dependent manner compared to utomilumab, based on IFN-gamma secretion.
FIG. 13b shows that FMC63-ZAAD01 dual antibody activates T cells in an EGFR-dependent manner compared to utomilumab, as analyzed for IFN-gamma secretion.

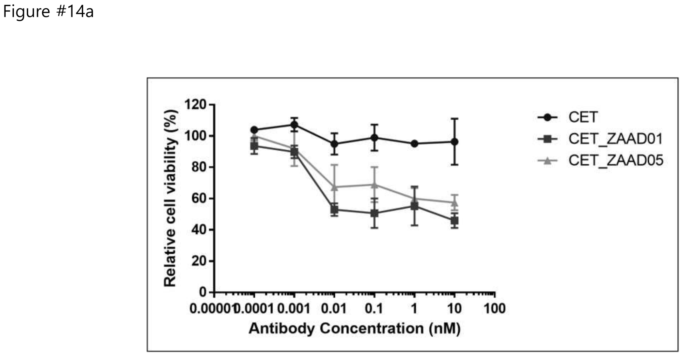

FIG. 14a shows that CET-ZAAD01 and CET-ZAAD05 induce immune cell-mediated killing of DLD1-luc cells.

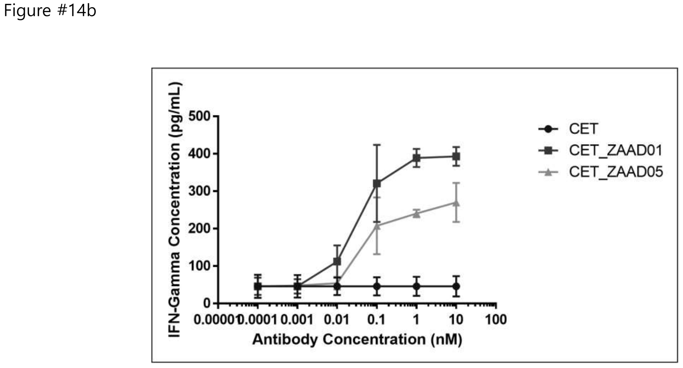

FIG. 14b shows that CET-ZAAD01 and CET-ZAAD05 increase IFN-gamma secretion via immune cell activation.

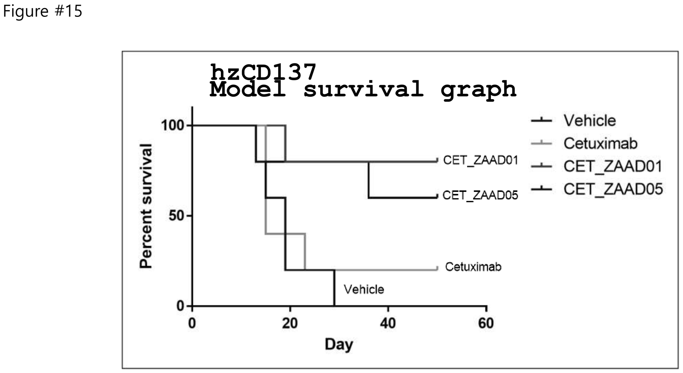

FIG. 15 shows increased survival in a humanized CD137 model as an anticancer effect of CET-ZAAD01 and CET-ZAAD05 bispecific antibodies.

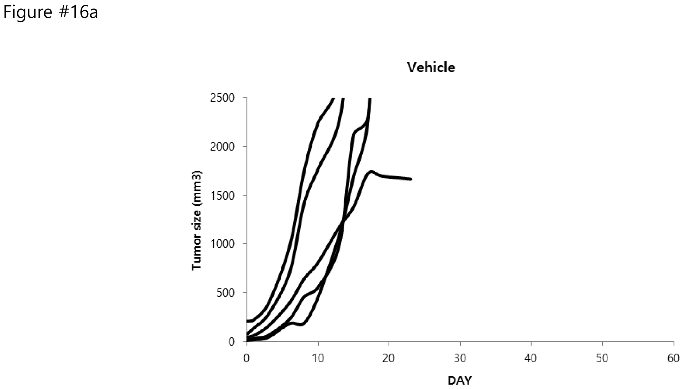

FIG. 16a shows tumor size in the vehicle-treated group in the humanized CD137 model.

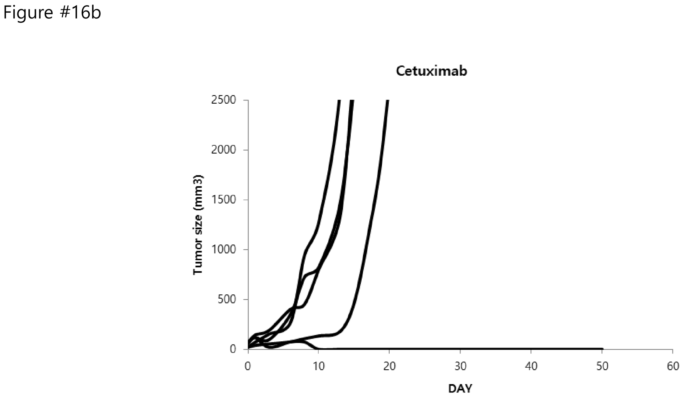

FIG. 16b shows tumor size in the cetuximab-treated group in the humanized CD137 model.

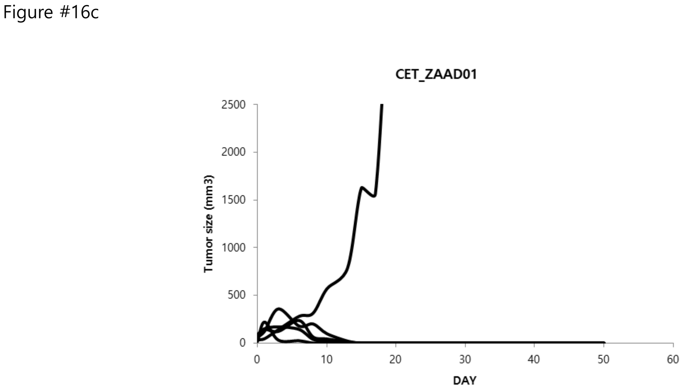

FIG. 16c shows tumor size in the CET-ZAAD01-treated group in the humanized CD137 model.

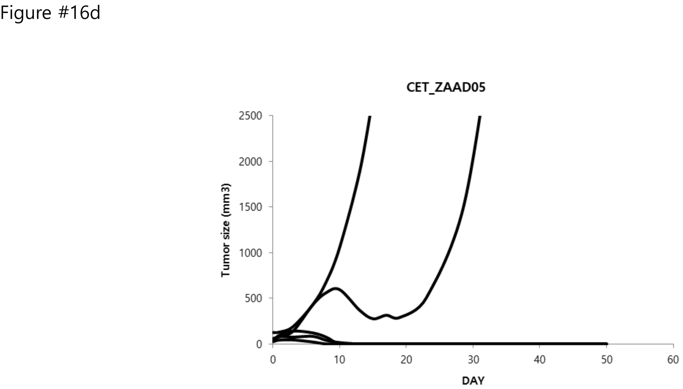

FIG. 16d shows tumor size in the CET-ZAAD05-treated group in the humanized CD137 model.

### Best Mode for Carrying out the Invention

A better understanding of the present disclosure may be obtained through the following examples, which are set forth to illustrate, but are not to be construed to limit, the scope of the present disclosure.

### EXAMPLES

Throughout the present specification, unless otherwise stated, the "%" used to indicate the concentration of a specific substance refers to (weight/weight)% for solid/solid, (weight/volume)% for solid/liquid, and (volume/volume)% for liquid/liquid.

### EXAMPLE 1. Development of Affibody Against CD137

### 1-1: Screening of Ten Affibody Clones Specifically Binding to Human CD137

Using CD137 protein, clones specifically binding to CD137 were selected from an affibody (Zb) library via panning. The selected clones were produced in the form of AffiMab (a bispecific antibody consisting of an affibody based on a monoclonal antibody) using mammalian cells, and clones that bound to CD137-expressing cells were further selected.

### Panning - Affinity Maturation

The affibody library was rescued into phage form using VSCM13 helper phage and used for panning. The number of library phages initially bound to the antigen was at least 10¹³, and the panning was conducted over four rounds. In successive rounds, the amount of antigen (10 µg, 5 µg, 2 µg, and 1 µg) was gradually reduced, and the number of washes (3, 5, 7, and 10 times) was increased to selectively enrich phages with high affinity. Binder phages obtained from each round were used to infect *E. coli* ER2537 (New England Biolabs ^{®} Inc.), and the resulting colonies were evaluated for antigen binding using ELISA.

### Periplasmic Extraction - Affibody Isolation

Colonies obtained by infecting with binder phages were cultured in SB medium (10 g/L MOPS, 20 g/L Bacto YEAST extract, and 30 g/L Trypton) until OD₆₀₀ (optical density at 600 nm) reached 0.8. Then, 1 mM IPTG (Isopropyl β-D-1-thiogalactopyranoside, LPS Solution Co., Ltd.) was added, followed by shaking incubation at 30°C to overexpress the affibody (Zb). Periplasmic extraction (PE) of affibody was carried out using BBS buffer (200 mM boric acid, 150 mM NaCl, and 1 mM EDTA), and binders were screened by ELISA using the extract.

### ELISA - Binder Screening

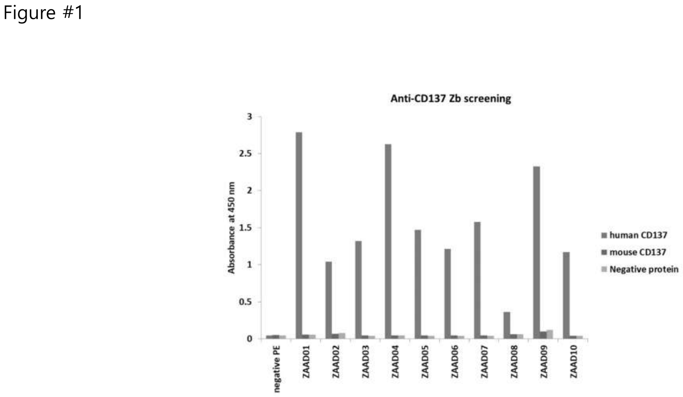

In the ELISA, plates coated with 2 µg/mL of hCD137-ECD-Fc (human CD137 extracellular domain-Fc fragment) protein were treated with affibody periplasmic extract. After treatment with a secondary antibody (anti-HA-HRP, Roche, 12013819001, human influenza hemagglutinin (HA) protein), color development was induced using TMB (3,3',5,5'-tetramethylbenzidine, BioFX^{™} TMBC-1000-01), and the OD₄₅₀ value was measured using an ELISA reader (Victor X3, PerkinElmer, Inc.). The results are depicted in FIG. 1. For comparison, plates coated with mouse CD137 ECD Fc protein or uncoated plates (negative protein) were also used. "Negative PE" refers to extract from *E. coli* ER2537 lacking the affibody plasmid.

### ELISA Results - Selection of Ten Affibody Clones

As shown in FIG. 1, little to no OD₄₅₀ value was measured for the negative PE or mouse CD137 protein, indicating that none of the ten affibody clones bound to them. In contrast, high OD₄₅₀ values were measured for all ten clones against the human CD137 protein, confirming their binding.

Based on the ELISA results, selection was made of ten affibodies binding to human CD137, designated ZAAD01 through ZAAD10. Sequence analysis of the affibodies confirmed that each of the ten clones was a unique clone (cloned specific cell line) specifically binding to human CD137.

### 1-2: Selection of Eight Affibody Clones

The ten unique clones described above were cloned in the form of Zb-Fc and produced using mammalian cells. Thereafter, binding (fusion) to CD137-expressing cells was confirmed. "Fc" refers to the fragment crystallizable region.

### T Cell Activation

CEMT cells (ATCC^{®}, CCL-119^{™}, T lymphoblast, acute lymphoblastic leukemia) were prepared at 1x10⁷ cells per 10 mL, and treated with 50 ng/mL of PMA (phorbol 12-myristate 13-acetate, Sigma-Aldrich^{®}, P1585) and 1 µg/mL of ionomycin (Sigma-Aldrich^{®}, I9657), followed by incubation for 16 hours to induce activation.

PMA and ionomycin are known to activate T cells. PMA activates protein kinase C, while ionomycin is a calcium ionophore. These two compounds pass through the T cell membrane receptor complex to induce activation of multiple intracellular signaling pathways and activate T cells, thereby inducing the production of various cytokines (see Ai, W., et al. (2013). Int J Environ Res Public Health. 2013; 10 (9): 3834-3842). Ionomycin, in combination with PMA, is known to stimulate the intracellular production of cytokines, interferons, perforins, IL-2, and IL-4. Furthermore, it is known that naive T cells, which are unstimulated, do not express CD137, whereas activated T cells express CD137 at transiently high levels upon stimulation.

The activated CEMT cells were prepared at 5x10⁵ cells per tube, then centrifuged at 1,200 rpm for 3 minutes to collect the cells, washed with PBS containing 5% FBS, and treated with the ten Zb-Fc proteins at a concentration of 5 µg/mL, followed by incubation on ice for 1 hour. The cells were washed three times by centrifugation at 1,200 rpm for 3 minutes using 200 µL of PBS containing 5% FBS. As a control, inactivated CEMT cells were also prepared.

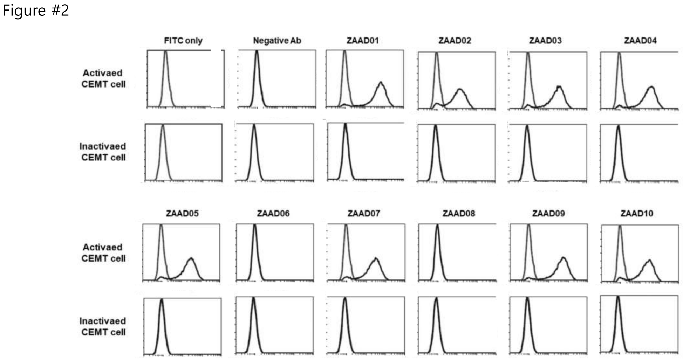

Subsequently, anti-human-Fc-FITC (Life Technologies^{®}, A11013) was added to the cells at 1 µg/mL, and the mixture was incubated on ice for 45 minutes in the dark. The cells were then washed three times by centrifugation at 1,200 rpm for 3 minutes using 200 µL of PBS containing 5% FBS. Fluorescence intensity was measured using a FACS (fluorescence-activated cell sorting) instrument from Beckman Coulter, Inc., and the results are shown in FIG. 2. In the FACS result graph, a peak shifted to the right indicates stronger binding to the cell. "FITC only" refers to the case where only buffer is present during Zb-Fc binding, and "Negative Ab" refers to the case using human IgG (hIgG).

As shown in FIG. 2, eight affibody clones - ZAAD01, ZAAD02, ZAAD03, ZAAD04, ZAAD05, ZAAD07, ZAAD09, and ZAAD10 - were selected as specifically binding to CD137-expressing cells, based on the FACS measurement results.

Specifically, the eight affibodies displayed only the FITC peak for inactivated CEMT cells, whereas for activated CEMT cells, they showed a peak shifted to the right of the FITC peak. This confirmed that the eight affibodies did not bind to inactivated CEMT cells, but bound specifically to activated CEMT cells.

However, the affibodies ZAAD06 and ZAAD08 did not show any peak shift between the negative Ab group (added with hIgG) and either inactivated or activated CEMT cells, indicating that they did not bind specifically to CD137-expressing (i.e., activated) CEMT cells. Therefore, ZAAD06 and ZAAD08 were excluded from selection.

### EXAMPLE 2. Selection of Affibodies Against CD137

### 2-1: Production of 16 Bispecific Antibodies

Based on two antibodies -cetuximab (CET) and a self-developed antibody, 15E3 (refer to Korean Patent No. 10-2017-0012754 A)- that specifically bind to epidermal growth factor receptor (EGFR), the eight CD137-binding affibodies selected in Example 1-2 were cloned in bispecific antibody formats. A total of 16 bispecific antibodies were produced using mammalian cells. When the eight affibodies were cloned in the form of bispecific antibodies with Cetuximab (CET), they were designated as CET-ZAAD01, CET-ZAAD02, CET-ZAAD03, CET-ZAAD04, CET-ZAAD05, CET-ZAAD07, CET-ZAAD09, and CET-ZAAD010, respectively. When cloned in the form of bispecific antibodies with the 15E3 antibody, they were designated as 15E3-ZAAD01, 15E3-ZAAD02, 15E3-ZAAD03, 15E3-ZAAD04, 15E3-ZAAD05, 15E3-ZAAD07, 15E3-ZAAD09, and 15E3-ZAAD010, respectively.

As disclosed in Korean Patent No. 10-2017-0012754 A, the inventors have previously confirmed that although both Cetuximab and the 15E3 antibody bind to domain 3 of EGFR, they bind to different epitopes.

### 2-2: Confirmation of Binding of Bispecific Antibodies to CD137 and EGFR Proteins

Using the purified bispecific antibody forms, CD137 affibody selection was conducted through binding tests to target proteins, binding to protein-expressing cells, and T cell activation tests, to identify affibodies that bind to both EGFR and CD137 proteins.

Binding of each of the 16 bispecific antibodies (8 Cetuximab-Zb and 8 15E3-Zb) produced in mammalian cells to the CD137 and EGFR proteins was confirmed by ELISA. In the ELISA, plates were coated with 2 µg/mL of hCD137-ECD-His and hEGFR-ECD-His proteins, respectively. The 16 purified bispecific antibodies were applied to the plates at seven dilution points, starting from 60 nM with 1:5 serial dilutions. After treatment with a secondary antibody (Goat anti-human IgG-F(ab')₂-HRP, Jackson ImmunoResearch Laboratories, Inc., JAC-109-035-097), color development was carried out using TMB (BioFX^{™} TMBC-1000-01), and the OD₄₅₀ values were measured using an ELISA reader (Victor X3, PerkinElmer, Inc.). EC₅₀ (half maximal effective concentration) values were calculated using GraphPad Prism software, and the results are shown in FIGS. 3a to 3d.

FIGS. 3a and 3b show the binding test results of bispecific antibodies comprising CD137 affibodies based on Cetuximab (CET) (hereinafter referred to as CET-based CD137 affibody bispecific antibodies, Cetuximab-Zb, or CET_ZAAD01 to CET_ZAAD10), and FIGS. 3c and 3d show the binding test results for bispecific antibodies comprising CD137 affibodies based on the 15E3 antibody (hereinafter referred to as 15E3-based CD137 affibody bispecific antibodies, 15E3-Zb, or 15E3_ZAAD01 to 15E3_ZAAD10). Specifically, FIGS. 3a and 3c show results of binding to EGFR protein, and FIGS. 3b and 3d show results of binding to CD137 protein.

From these results, it was confirmed that all the 16 bispecific antibodies of the present disclosure - both CET-based and 15E3-based CD137 affibody bispecific antibodies - bind to both EGFR and CD137 proteins.

### 2-3: Confirmation of Binding of Bispecific Antibodies to CD137-Expressing Cells

To confirm the binding of the 16 bispecific antibodies to CD137-expressing cells, CEMT cells were prepared at 1×10⁷ cells/10 mL and activated by incubation with 50 ng/mL of PMA and 1 µg/mL of ionomycin for 16 hours. The activated CEMT cells were then prepared at 5×10⁵ cells/tube, harvested by centrifugation at 1,200 rpm for 3 minutes, washed with PBS containing 5% FBS, treated with each bispecific antibody at 5 µg/mL, and incubated on ice for 1 hour. The cells were washed three times by centrifugation at 1,200 rpm for 3 minutes using 200 µL of PBS supplemented with 5% FBS. Then, anti-human-Fc-FITC (Life Technologies^{®}, A11013) was added at 1 µg/mL, and the cells were incubated in the dark on ice for 45 minutes. After three additional washes in the same manner, fluorescence intensity was measured using a FACS (fluorescence-activated cell sorting) instrument from Beckman Coulter, and the results were shown in FIGS. 4a and 4b. As a positive control (Positive Ab), Utomilumab was used while human IgG (hIgG) was used as a negative control.

FIG. 4a shows the FACS results for CET-based CD137 affibody bispecific antibodies. Like the peak of the positive control, the peaks were shifted to the right, confirming that all eight CET-based CD137 affibody bispecific antibodies bound to activated CEMT cells expressing CD137.

FIG. 4b shows the FACS results for 15E3-based CD137 affibody bispecific antibodies. Similarly, as the peaks were shifted to the right, like the positive control, all eight 15E3-based CD137 affibody bispecific antibodies were confirmed to bind to activated CEMT cells expressing CD137.

From these results, it was confirmed that all 16 bispecific antibodies of the present disclosure specifically bind to CD137-expressing cells.

### 2-4: Confirmation of Binding of Bispecific Antibodies to EGFR-Expressing Cells

Likewise, to confirm the binding of the 16 bispecific antibodies to EGFR-expressing cells, HT29 cells (Korean Cell Line Bank, KCLB, 30038, a human colorectal cancer cell line), known to highly express EGFR, were prepared at 5×10⁵ cells/tube and harvested by centrifugation at 1,200 rpm for 3 minutes. After washing with PBS containing 5% FBS, the 16 bispecific antibodies were treated at a concentration of 5 µg/mL and incubated on ice for 1 hour. The cells were washed three times by centrifugation at 1,200 rpm for 3 minutes using 200 µL of PBS containing 5% FBS. Then, anti-human-Fc-FITC (Life Technologies^{®}, A11013) was added at 1 µg/mL, and the cells were incubated in the dark on ice for 45 minutes. After three additional washes, fluorescence intensity was measured using a FACS instrument from Beckman Coulter, and the results were shown in FIGS. 5a and 5b. For positive controls, CET-IgG was used in CET-based evaluation, and 15E3-IgG was used in 15E3-based evaluation; human IgG (hIgG) was used as the negative control.

FIG. 5a shows the FACS results for CET-based CD137 affibody bispecific antibodies. The peaks were shifted to the right, similar to the positive control (CET-IgG), confirming that all eight CET-based CD137 bispecific antibodies bound to EGFR-expressing HT29 cells.

FIG. 5b shows the FACS results for 15E3-based CD137 affibody bispecific antibodies. The peaks were also shifted to the right, like the positive control (15E3-IgG), confirming that all eight 15E3-based CD137 bispecific antibodies bound to EGFR-expressing HT29 cells.

From these results, it was confirmed that all 16 bispecific antibodies of the present disclosure specifically bind to EGFR-expressing cells.

### 2-5: EGFR-Dependent T Cell Activation Test - Selection of Affibodies ZAAD01 and ZAAD05

From the total 16 bispecific antibodies produced, affibody selection was conducted through an EGFR-dependent T cell activation test.

### Test Method

One day prior to the test, 50 µL of anti-CD3 antibody (Invitrogen^{™}, 16-0037-85) was coated on a 96-well round-bottom plate at a concentration of 3 µg/mL. On the following day, the wells were washed three times using 100 µL of sterile PBS. Then, 50 µL of 5 µg/mL hEGFR-ECD-Fc was coated again, and for the control group, PBS was applied to wells without protein. The plate was incubated at 37°C for 3 hours. The wells were washed three times with 100 µL of R10 medium (RPMI1640, 10% FBS, and 10 mM HEPES), then blocked with 100 µL of R10 medium for 1 hour at 37°C. After complete removal of R10 medium, 50 µL of each of the 16 bispecific antibodies (8 Cetuximab-Zb and 8 15E3-Zb) was applied at concentrations of 0.1 nM or 1 nM for 30 minutes.

Blood from two donors was diluted 1:2 with PBS and layered on Leucosep tubes (Greiner Bio-One, Cat. No. 227290) pre-filled with Ficoll^{®} (GE Healthcare, 17-1440-12), followed by centrifugation at 1,000 ×g for 30 minutes. Only the layer of peripheral blood mononuclear cells (PBMCs) above the Ficoll was collected. The PBMCs were washed twice by centrifugation at 300 ×g for 10 minutes using PBS with 2% FBS, and the number of PBMCs was counted. CD8⁺ T cells were isolated from the PBMCs according to the protocol of the CD8⁺ T Cell Isolation Kit (Miltenyi Biotec Inc., 130-096-495).

The number of CD8⁺ T cells was adjusted to 1.4 × 10⁶ cells/mL using R10 medium, and 50 µL of the suspension was added to each well containing the 16 bispecific antibodies. After 3 days of incubation, the plate was centrifuged at 2,000 rpm for 10 minutes, and the supernatant was collected. Interferon gamma (IFN-γ) levels were measured using the Human IFN-gamma ELISA Set (BD Biosciences, 555142), following the manufacturer's protocol. The results are shown in FIG. 6.

### Test Results

As shown in FIG. 6, T cell activation was assessed by measuring IFN-γ levels in the presence or absence of EGFR. The results demonstrated that treatment with CET-based ZAAD01 and ZAAD05 bispecific antibodies, and 15E3-based ZAAD01 and ZAAD05 bispecific antibodies at 1 nM concentration significantly increased IFN-γ levels. Therefore, among the eight affibodies tested, ZAAD01 and ZAAD05 were selected based on their ability to increase IFN-γ production.

Specifically, in the EGFR-dependent T cell activation test, Cetuximab-Zb or 15E3-Zb binds to hEGFR-ECD-Fc coated on the plate, and CD8⁺ T cells expressing CD3 bind to the anti-CD3 antibody coated on the plate. The affibody portion of Cetuximab-Zb or 15E3-Zb that is bound to hEGFR-ECD-Fc then binds to CD137 on the surface of the CD8⁺ T cell attached to the anti-CD3 antibody, thereby activating the CD8⁺ T cell, which subsequently secretes IFN-γ. It is known that CD137 agonism activates T cells.

The affibodies ZAAD01 and ZAAD05 of the present disclosure activated CD137 by binding to the CD137 extracellular domain (ECD) on the surface of CD8⁺ T cells, leading to IFN-γ secretion by the CD8⁺ T cells.

These results are consistent with prior literature reporting that the monoclonal antibodies Urelumab (BMS-663513) and Utomilumab act as agonists by binding to CD137 (4-1BB) and activating T cells (see U.S. Patent No. US7288638 B2, Oct. 30, 2007;U.S. Patent No. 7288638 B2; and U.S. Patent No. US8821867 B2).

Based on these results, the inventors confirmed that the bispecific antibodies Cetuximab-ZAAD01, 15E3-ZAAD01, Cetuximab-ZAAD01, and 15E3-ZAAD01 activated T cells in an EGFR-dependent manner, and ultimately selected affibodies ZAAD01 and ZAAD05.

### Example 3. Characterization of Selected Affibodies

### 3-1: Analysis of Affibody Binding Properties via CD137 ECD Domain Mapping

The binding characteristics of the two selected affibodies, ZAAD01 and ZAAD05, were confirmed through domain mapping of CD137. Hereinafter, the 15E3-based ZAAD01 and ZAAD05 affibody bispecific antibodies are referred to as 15E3ZAAD01 and 15E3ZAAD05, respectively. The binding domains of the monoclonal antibodies Urelumab (BMS-663513) and Utomilumab (PF-05082566), which also target CD137, were likewise analyzed.

To determine which region of the extracellular domain (ECD) of CD137 the selected CD137 affibodies bind to, the ECD of CD137 was divided into five domains, and domain mutants were cloned. A schematic of the domain mutants is shown in FIG. 7a.

As shown in FIG. 7a, the cloned domain mutants include the signal peptide and various combinations of ECD domains 1 to 5, specifically: hCD137-ECD (containing domains 1 to 5 and the signal peptide); hCD137-ECD-ΔD1 (domain 1 deleted); hCD137-ECD-ΔD12 (domains 1 and 2 deleted); hCD137-ECD-ΔD123p (domains 1 and 2 and part of domain 3 (up to amino acid 104) deleted); hCD137-ECD-ΔD123 (domains 1 to 3 deleted); and hCD137-ECD-ΔD3 and hCD137-ECD-ΔD5 (domain 3 or domain 5 deleted, respectively).

FIG. 7a presents a schematic diagram of hCD137-ECD including the signal peptide and domains 1 to 5. SEQ ID NO: 27 shows the amino acid sequence of domains 1 to 5, excluding the signal peptide.

The cloned domain mutants were expressed in mammalian cells. ELISA plates were coated with the expressed domain mutants at 2 µg/mL, and purified Urelumab, Utomilumab, 15E3, 15E3ZAAD01, and 15E3ZAAD05 were applied at concentrations of 10 µg/mL, 2 µg/mL, and 0.4 µg/mL. After applying a secondary antibody (anti-hIgG-Fab-HRP, Jackson ImmunoResearch Laboratories, Inc., JAC-109-035-097), a color reaction was developed using TMB (BioFX^{™} TMBC-1000-01), and OD₄₅₀ values were measured using an ELISA reader (Victor X3, PerkinElmer, Inc.). The results are shown in FIGS. 7b through 7f.

As shown in FIG. 7b, Urelumab did not bind to hCD137-ECD-ΔD1, hCD137-ECD-ΔD12, hCD137-ECD-ΔD123p, or hCD137-ECD-ΔD123, indicating that Urelumab binds to domain 1 of CD137 ECD.

As shown in FIG. 7c, Utomilumab did not bind to hCD137-ECD-ΔD123p, hCD137-ECD-ΔD123, or hCD137-ECD-ΔD3, indicating that Utomilumab binds to domain 3 of CD137 ECD.

As shown in FIG. 7d, the negative Ab (Cetuximab) did not bind to full-length human CD137-ECD or any of its domain mutants, confirming no binding to CD137 ECD.

As shown in FIG. 7e, 15E3ZAAD01 did not bind to hCD137-ECD-ΔD123p, hCD137-ECD-ΔD123, or hCD137-ECD-ΔD3, indicating that 15E3ZAAD01 binds to domain 3 of CD137 ECD.

As shown in FIG. 7f, 15E3ZAAD05 also did not bind to hCD137-ECD-ΔD123p, hCD137-ECD-ΔD123, or hCD137-ECD-ΔD3, indicating that 15E3ZAAD05 binds to domain 3 of CD137 ECD.

Based on these results, the inventors confirmed that the two selected affibodies, ZAAD01 and ZAAD05, bind to domain 3 of the CD137 ECD.

### 3-2: Cross-Reactivity of Selected Affibodies with the TNFRSF

The cross-reactivity of the two selected affibodies, ZAAD01 and ZAAD05, with members of the tumor necrosis factor receptor superfamily (TNFRSF) was tested using ELISA.

ELISA plates were coated at 2 µg/mL with proteins from the TNFRSF family, including CD137 (TNFRSF9), CD27 (TNFRSF7), CD30 (TNFRSF8), CD134 (TNFRSF4), CD270 (TNFRSF14), CD357 (TNFRSF18), and TNF-alpha. 15E3ZAAD01 and 15E3ZAAD05 were applied at 1 µg/mL, followed by treatment with a secondary antibody (anti-hIgG-Fab-HRP, Jackson ImmunoResearch Laboratories, Inc., JAC-109-035-097). Color development was performed using TMB (BioFX^{™} TMBC-1000-01), and OD₄₅₀ values were measured using an ELISA reader (Victor X3, PerkinElmer, Inc.). The results are shown in FIGS. 8a and 8b.

As shown in FIG. 8a, ZAAD01 specifically bound only to CD137, and as shown in FIG. 8b, ZAAD05 also specifically bound only to CD137.

From these results, the inventors confirmed that the selected two affibodies, ZAAD01 and ZAAD05, did not cross-react with other TNFRSF proteins.

### 3-3: Species Cross-Reactivity of Selected Affibodies

The species cross-reactivity of the two selected affibodies, ZAAD01 and ZAAD05, to CD137 was tested using ELISA.

ELISA plates were coated at 2 µg/mL with human CD137, mouse CD137, and cynomolgus monkey CD137 proteins. 15E3ZAAD01 and 15E3ZAAD05 were treated at 5 µg/mL. After applying a secondary antibody (anti-hIgG-Fab-HRP, Jackson ImmunoResearch Laboratories, Inc., JAC-109-035-097), color development was induced using TMB (BioFX^{™} TMBC-1000-01), and OD₄₅₀ values were measured using an ELISA reader (Victor X3, PerkinElmer, Inc.). The results are shown in FIGs. 9a and 9b.

As shown in FIG. 9a,the affibody ZAAD01 bound to CD137 proteins from both humans and cynomolgus monkeys, and as shown in FIG. 9b, the affibody ZAAD05 also bound to CD137 proteins from both humans and cynomolgus monkeys.

From these results, it was confirmed that both selected CD137 affibodies exhibited species cross-reactivity with CD137 in humans and cynomolgus monkeys.

### EXAMPLE 4. Binding of Bispecific Antibodies to Target Proteins and EGFR-Dependent Activity

### 4.1: Confirmation of Simultaneous Binding of Bispecific Antibodies to Two Targets

Whether the four bispecific antibodies - CET-ZAAD01, CET-ZAAD05, 15E3-ZAAD01, and 15E3-ZAAD05 - composed of anti-EGFR antibodies Cetuximab and 15E3, and CD137-binding affibodies ZAAD01 and ZAAD05, simultaneously bind to each target protein was confirmed using BioLayer Interferometry (BLI).

EGFR-ECD-Fc protein was immobilized on an AR2G sensor chip (Amine Reactive Second-Generation Biosensor, SARTORIUS, Cat. No. 18-5092) at a concentration of 5 µg/mL using EDC/NHS amine coupling. The four bispecific antibodies were each applied at 10 µg/mL to the sensor chip pre-coated with EGFR-ECD-Fc and allowed to bind for 5 minutes, followed by 5 minutes of stabilization. Then, CD137-ECD-his protein was added at 30 µg/mL to the sensor chip having the dual antibody bound to the immobilized EGFR protein and allowed to bind for 5 minutes, followed by another 5-minute stabilization period. The results are shown in FIGS. 10a to 10d. As controls, treatment with CET or 15E3 alone is indicated by red lines, and treatment with the four bispecific antibodies of the present disclosure is indicated by blue lines.

As shown in FIG. 10a, when CET was applied as a control and CD137-ECD-Fc protein was added to the sensor chip, there was no change in the binding signal (red line) . In contrast, when CET-ZAAD01 bispecific antibody was applied, the binding signal increased upon addition of CD137-ECD-Fc protein (blue line), indicating that the CET-ZAAD01 bispecific antibody simultaneously binds to the EGFR protein immobilized to the sensor chip and the subsequently added CD137-ECD-Fc protein.

Similarly to the CET-ZAAD01 bispecific antibody, CET-ZAAD05 bispecific antibody (FIG. 10b), 15E3-ZAAD01 bispecific antibody (FIG. 10c), and 15E3-ZAAD05 bispecific antibody (FIG. 10d) also showed increased binding signals upon addition of CD137-ECD-his protein to the sensor chip, confirming that these bispecific antibodies also bind simultaneously to both EGFR and CD137 proteins.

### 4.2: T Cell Activation by Bispecific Antibodies Depending on EGFR Expression Levels

It was investigated whether bispecific antibodies composed of CD137-binding affibodies and anti-EGFR antibodies could enhance T cell activation depending on the level of EGFR expression in various cell lines.

### EGFR Expression Levels in Cell Lines

Three EGFR-expressing cell lines A431 (ATCC, CRL-1555, epidermoid carcinoma), HT29 (KCLB, 30038, colon adenocarcinoma), and SW403 (KCLB, 10230, colon adenocarcinoma) and one EGFR-negative cell line MOLT4 (KCLB, 21582, adult T acute lymphoblastic leukemia) were each prepared at 5x10⁵ cells per tube.

The cells were harvested by centrifugation at 1,200 rpm for 3 minutes, washed with PBS containing 5% FBS, and treated with the 15E3ZAAD01 bispecific antibody at 5 µg/mL. After incubation on ice for 1 hour, the cells were washed three times using 200 µL of PBS with 5% FBS and centrifugation at 1,200 rpm for 3 minutes. Then, antihuman Fc FITC was applied at 1 µg/mL, followed by incubation in the dark on ice for 45 minutes. After another three washes using the same method, fluorescence intensity was measured using a FACS instrument from Beckman Coulter, and the mean fluorescence intensity (MFI) values were obtained. The results are shown in FIG. 11a. The higher the binding of the 15E3ZAAD01 bispecific antibody to the cells, the higher the MFI value, which indicates a higher level of EGFR expression.

As shown in FIG. 11a, EGFR expression was evaluated in the EGFR-positive cell lines A431, HT29, and SW403, as well as the EGFR-negative MOLT4 cell line. The MFI values were 101.5, 23.9, 17.1, and 0.9 for A431, HT29, SW403, and MOLT4, respectively, confirming that EGFR expression levels followed the same order.

### EGFR-Dependent T Cell Activation by Bispecific Antibody

Using cell lines with different levels of EGFR expression as examined above, a T cell assay was conducted. One day prior to the assay, anti-CD3 antibody (Invitrogen^{™}, 16-0037-85) was coated onto a 96-well round-bottom plate at 3 µg/mL in 50 µL per well. The next day, the wells were washed three times with 100 µL of sterile PBS. Then, the wells were washed three times with 100 µL of R10 medium, followed by blocking with 100 µL of R10 medium at 37°C for 1 hour. The four cell lines -A431, HT29, SW403, and MOLT4- were added at a concentration of 2.4×10⁵ cells/mL in 50 µL per well. The 15E3ZAAD01 bispecific antibody was then added at a concentration of 5 µg/mL in 50 µL and incubated for 30 minutes.

Meanwhile, blood obtained from two donors was diluted 1:2 with PBS and layered into Leucosep tubes (Greiner Bio-One, Cat. No. 227290) pre-filled with Ficoll^{®} (GE Healthcare, 17-1440-12), followed by centrifugation at 1,000 ×g for 30 minutes.

Only the PBMC layer above the Ficoll was collected. The PBMCs were washed twice by centrifugation at 300 ×g for 10 minutes using PBS with 2% FBS, and the PBMC was counted. CD8⁺ T cells were prepared from PBMC according to the protocol of the CD8⁺ T Cell Isolation Kit (Miltenyi Biotec Inc., 130-096-495).

The number of CD8⁺ T cells was adjusted to 1.4×10⁶ cells/mL using R10 medium, and 50 µL of the cell suspension was added to the wells containing the 15E3ZAAD01 bispecific antibody-treated cells. After 3 days, the plate was centrifuged at 2,000 rpm for 10 minutes, and the supernatant was collected. IFN-γ levels were measured using an IFN-γ assay kit (BD Biosciences, 555142) according to the manufacturer's protocol. Whether the bispecific antibody of the present disclosure activates T cells in an EGFR expression-dependent manner was evaluated by measuring IFN-γ secretion from T cells. The results are shown in FIG. 11b.

As shown in FIG. 11b, T cell activation analysis revealed that IFN-γ levels increased with increasing EGFR expression, confirming that T cell activation also increased with EGFR expression.

Specifically, the order of IFN-γ secretion levels corresponded to the order of EGFR expression levels described earlier. The IFN-γ fold-change values were 5.9, 2.2, 1.9, and 1.0 for cell lines A431, HT29, SW403, and MOLT4, respectively, confirming that IFN-γ secretion increased in that order.

Based on these results, the inventors confirmed EGFR-dependent T cell activation by the 15E3-ZAAD01 bispecific antibody according to EGFR expression level, which indicates that the degree of T cell activation correlates with the level of EGFR expression.

When using the EGFR antibody-based CD137 affibody bispecific antibodies of the present disclosure, CD137-expressing T cells can bind and become activated in close proximity to tumor cells or tissues with high EGFR overexpression. Therefore, the antibodies can be usefully applied for the prevention or treatment of various EGFR-overexpressing cancers.

### EXAMPLE 5. Potential for Development of CD137 Affibody-Based Bispecific Antibodies Targeting Various Antigens - HER2 and CD19

Based on the results described above, the inventors sought to determine whether CD137 affibody-based bispecific antibodies using antibodies against other cancer- or tumor-specific target proteins, in addition to EGFR as a cancer-specific targeting protein, could activate T cells.

### 5.1: Confirmation of Binding to Target Proteins HER2 or CD19

Using the selected CD137-binding affibody ZAAD01 as a base, bispecific antibodies were cloned using antibodies against HER2 and CD19-proteins other than EGFR. The bispecific antibodies composed of a HER2 antibody and the CD137 affibody were designated as anti-HER2 Ab1-ZAAD01 and anti-HER2 Ab2-ZAAD01. The bispecific antibody composed of a CD19 antibody and the CD137 affibody was designated as anti-CD19 Ab-ZAAD01.

Specifically, in anti-HER2 Ab1-ZAAD01, anti-HER2 Ab1 is trastuzumab, which specifically binds to human HER2. In anti-HER2 Ab2-ZAAD01, anti-HER2 Ab2 is a self-developed antibody hz1E11.10 that specifically binds to human HER2 (see Korean Patent No. 10-2017-0117330 A).

The anti-HER2 Ab may be an antibody or an antigen-binding fragment thereof composed of a heavy chain variable region including the amino acid sequence of SEQ ID NO: 28, and a light chain variable region including the amino acid sequence of SEQ ID NO: 29.

Specifically, in anti-CD19 Ab-ZAAD01, the anti-CD19 Ab is FMC63, which specifically binds to human CD19.

The anti-CD19 Ab may be an antibody or an antigen-binding fragment thereof composed of a heavy chain variable region including the amino acid sequence of SEQ ID NO: 38, and a light chain variable region including the amino acid sequence of SEQ ID NO: 39.

The above-described bispecific antibodies were produced using mammalian cells. Using the purified bispecific antibody forms, binding to the proteins and T cell activation tests were performed to examine the feasibility of using the CD137 affibody of the present disclosure in a modular format for bispecific antibodies targeting various other antigens.

ELISA was performed by coating plates with CD137 protein and the respective target proteins (hHER2-ECD-Fc and hCD19-ECD-Fc) at a concentration of 2 µg/mL. The purified bispecific antibodies described above were applied in seven dilution points starting from 60 nM (1:5 serial dilution). A secondary antibody (anti-hIgG-Fab-HRP, Jackson ImmunoResearch Laboratories, Inc., JAC-109-035-097) was applied, followed by color development with TMB (BioFX^{™} TMBC-1000-01). OD₄₅₀ values were measured using an ELISA reader (Victor X3, PerkinElmer, Inc.), and the results are shown in FIGS. 12a to 12d.

As shown in FIGS. 12a to 12d, the bispecific antibodies comprising CD137 affibody and HER2 or CD19 antibodies bound to both CD137 and HER2 or CD19, respectively.

Specifically, compared to the negative control (hIgG), both anti-HER2 Ab1-ZAAD01 and anti-HER2 Ab2-ZAAD01-bispecific antibodies composed of anti-HER2 antibodies and ZAAD01 affibody-showed concentration-dependent increases in binding to the target proteins CD137 (FIG. 12a) and HER2 (FIG. 12b).

In addition, compared to the negative control, the anti-CD19 Ab-ZAAD01 bispecific antibody-composed of an anti-CD19 antibody and ZAAD01 affibody-showed concentration-dependent increases in binding to the target proteins CD137 (FIG. 12c) and CD19 (FIG. 12d).

From these results, it was confirmed that the bispecific antibodies anti-HER2 Ab1-ZAAD01 and anti-HER2 Ab2-ZAAD01 of the present disclosure bind to both CD137 and HER2 proteins, and that the bispecific antibody anti-CD19 Ab-ZAAD01 binds to both CD137 and CD19 proteins.

### 5.2: Target Protein-Dependent T Cell Activation Test

As confirmed above, the bispecific antibodies bound to both target proteins. T cell activation tests were performed to evaluate the efficacy of the bispecific antibodies, specifically assessing whether activation occurred in a HER2- or CD19-dependent manner.

### Test Method

One day prior to the test, anti-CD3 antibody (Invitrogen, 16-0037-85) was coated on a 96-well round-bottom plate at a concentration of 3 µg/mL in 50 µL per well. On the following day, the wells were washed three times using 100 µL of sterile PBS. Then, 50 µL of either HER2 or CD19 protein at a concentration of 5 µg/mL was coated again, and for the control wells (not treated with HER2 or CD19), only PBS was added. The plate was incubated at 37°C for 3 hours. The wells were then washed three times with 100 µL of R10 medium, followed by blocking with 100 µL of R10 medium for 1 hour at 37°C. After completely removing the R10 medium, the bispecific antibodies were applied at a concentration of 5 µg/mL in 50 µL and incubated for 30 minutes.

Meanwhile, whole blood collected from two donors was diluted 1:2 with PBS and layered into Leucosep tubes (Greiner Bio-One, Cat. No. 227290) pre-filled with Ficoll^{®} (GE Healthcare, 17-1440-12), followed by centrifugation at 1,000 ×g for 30 minutes. Only the PBMC (peripheral blood mononuclear cell) layer above the Ficoll was collected. The PBMCs were washed twice by centrifugation at 300 ×g for 10 minutes using PBS with 2% FBS, and the PBMC were counted.

CD8⁺ T cells were isolated from the PBMCs using the CD8⁺ T Cell Isolation Kit (Miltenyi Biotec Inc., 130-096-495) according to the manufacturer's protocol. The number of CD8⁺ T cells was adjusted to 1.4×10⁶ cells/mL using R10 medium, and 50 µL of the suspension was added to the wells pre-treated with the bispecific antibodies. After 3 days of incubation, the plate was centrifuged at 2,000 rpm for 10 minutes, and the supernatant was collected. IFN-γ levels were measured using an IFN-γ ELISA kit (BD Biosciences, 555142) according to the manufacturer's protocol. The results are shown in FIGs. 13a and 13b. Utomilumab, a monoclonal antibody against CD137, was used as a negative control.

### Test Results

As shown in FIGS. 13a and 13b, the bispecific antibodies produced using the CD137 affibody in combination with HER2 or CD19 antibodies (anti-HER2 Ab1-ZAAD1, anti-HER2 Ab2-ZAAD1, and anti-CD19 Ab-ZAAD1) activated T cells in a HER2- or CD19-dependent manner, as confirmed by increased IFN-γ secretion.

Specifically, in FIG. 13a, when the monoclonal antibody Utomilumab against CD137 was administered, IFN-γ was scarcely secreted from CD8⁺ T cells regardless of the presence of HER2. In contrast, when the bispecific antibodies anti-HER2 Ab1-ZAAD01 (trastuzumab-ZAAD01) and anti-HER2 Ab2-ZAAD01 (hz1E11.10-ZAAD01) were administered, CD8⁺ T cells secreted significantly more IFN-γ in the presence of HER2 compared to the absence of HER2.

Likewise, in FIG. 13b, treatment with Utomilumab, which is a monoclonal antibody to CD137, resulted in no significant difference in IFN-γ secretion by CD8⁺ T cells regardless of CD19 presence. However, when the bispecific antibody anti-CD19 Ab-ZAAD01 (FMC63-ZAAD01) was administered, CD8⁺ T cells secreted significantly more IFN-γ in the presence of CD19 compared to the absence of CD19.

From the IFN-γ secretion analysis, the inventors confirmed that the bispecific antibodies composed of either an anti-HER2 or anti-CD19 antibody and the ZAAD01 affibody activated T cells in a target protein-dependent manner, specifically in the presence of HER2 or CD19, respectively.

In particular, these results demonstrate that T cell activation, depending on the additional target proteins HER2 or CD19, was confirmed by measuring IFN-γ. This suggests that when the CD137 affibody of the present disclosure is used in a modular format, it can be applied not only to the target proteins disclosed herein (EGFR, HER2, and CD19), but also to various other target proteins.

Accordingly, those skilled in the art will understand from the disclosure of the present invention that a bispecific antibody composed of an antibody targeting a protein specifically expressed or present in certain cancer cells and the CD137 affibody of the present disclosure can be usefully applied for the prevention or treatment of various types of cancer via immunomodulatory approaches involving CD137.

Ultimately, the CD137 affibody of the present disclosure, or a bispecific antibody composed of the CD137 affibody and an antibody against a cancer cell-specific target protein, may be effectively used for the prevention or treatment of various types of cancer through CD137-expressing antigen-presenting cells (APCs), such as NK (natural killer) cell-mediated ADCC (antibody-dependent cell-mediated cytotoxicity) or dendritic cell-mediated T cell activation.

### Example 6. Confirmation of T Cell Activation and Cell Death Induced by EGFR-CD137-Targeting Bispecific Antibodies

The present inventors conducted the following experiment to confirm the T cell activation and cell-killing effect of bispecific antibodies simultaneously targeting EGFR and CD137. The bispecific antibodies (CET_ZAAD01 and CET_ZAAD05) used in this Example are fusion proteins consisting of Cetuximab, which targets EGFR, and the affibody ZAAD01 or ZAAD05 of the present disclosure, which targets CD137.

One day prior to the bispecific antibody treatment, an anti-CD3 antibody (Invitrogen, 16-0037-85) was coated on a 96-well round plate at a concentration of 5 µg/mL in 50 µL per well. On the following day, 100 µL of R10 medium was added, followed by incubation at 37°C for 1 hour for blocking. DLD-1-Luc cells were added to each well at 50 µL per well to a final concentration of 1.0 × 10⁵/mL. The bispecific antibodies (CET _ZAAD01 and CET-ZAAD05), prepared in serial 10-fold dilutions starting at 10 nM, were added to each well in 50 µL volumes. Meanwhile, blood from a donor was diluted 1:2 with PBS, layered onto Leucosep tubes (Greiner Bio-One, 227290) containing Ficoll (GE Healthcare, 17-1440-12), and centrifuged at 1,000 × g for 30 minutes. The PBMC layer above the Ficoll was collected, mixed with 40 mL of PBS containing 2% FBS, and washed twice by centrifugation at 300 x g for 10 minutes. The number of PBMCs was then counted. The PBMCs were resuspended in R10 medium to a concentration of 1.0 × 10⁵/mL and added to the wells containing the bispecific antibodies in 50 µL volumes. After 3 days of incubation, the plate was centrifuged at 2,000 rpm for 10 minutes, and the supernatant was collected to measure the amount of IFN-γ using an IFN-γ assay kit (BD, 555142). To the wells containing the remaining cells and media, 50 µL of lysis buffer (75 mM Tris [pH 8.0], 30% glycerol, 3% Triton X-100) was added and mixed well, followed by incubation at room temperature for 15 minutes. Then, 50 µL of the mixture of cells and lysis buffer was transferred to a 96-well white plate, and an equal volume of 5'-fluoroluciferin reagent (Bio-Glo^{™} Luciferase Assay System, Promega, G7940) was added. After incubating at room temperature for 15 minutes, luminescence was measured using an ELISA reader.

The results are shown in FIGS. 14a and 14b.

As shown in FIGS. 14a and 14b, the bispecific antibodies induced activation of immune cells, resulting in the death of DLD1-Luc cells and a concurrent increase in the concentration of IFN-γ.

### EXAMPLE 7: Evaluation of Antitumor Efficacy of EGFR-CD137-Targeting Bispecific Antibodies Using an Animal Model

The efficacy of bispecific antibodies targeting EGFR and CD137 (CET_ZAAD01 and CET-ZAAD05) was evaluated using a humanized CD137 mouse model.

EGFR/MC38 cells were prepared in PBS at a concentration of 5.0 × 10⁵/mL and subcutaneously injected into the flank of humanized CD137 mice to artificially induce tumor formation. Once the tumor volume reached approximately 100 mm³, mice were randomly grouped into sets of five as per the experimental design. Cetuximab or the EGFR-CD137-targeting bispecific antibodies (CET_ZAAD01 and CET-ZAAD05) were diluted in PBS to a concentration of 5 mpk (mg/kg) and administered intraperitoneally twice a week for a total of five doses. Tumor size and weight were measured three times a week, and tumor size was determined using a digital vernier caliper. Mice were euthanized if the tumor size exceeded 2500 mm³.

The results are shown in FIG. 15 and FIGS. 16a through 16d.

As shown in FIG. 15, the two bispecific antibodies of the present disclosure (CET_ZAAD01 and CET-ZAAD05) prolonged the survival of the mice.

Furthermore, as shown in FIGS. 16a to 16d, the bispecific antibodies of the present disclosure were confirmed to reduce tumor size.

In contrast, in the vehicle-treated and Cetuximab-treated groups, tumor size continued to increase, whereas in the two bispecific antibody-treated groups (CET _ZAAD01 and CET-ZAAD05), tumor growth was suppressed, and tumor cell death was observed.

While specific aspects of the present disclosure have been described in detail above, it will be apparent to those skilled in the art that such specific descriptions are merely preferred embodiments, and the scope of the present disclosure is not limited thereby.

## Claims

1. An affibody, comprising an amino acid sequence represented by any one of SEQ ID NOS: 1 to 8, which specifically binds to an extracellular domain of CD137.

2. The affibody of claim 1, wherein the CD137 is derived from a human or cynomolgus monkey.

3. The affibody of claim 1, wherein the binding site to the extracellular domain of CD137 is located between the 64th and 95th amino acid residues of the amino acid sequence of SEQ ID NO: 27.

4. A nucleic acid molecule comprising a nucleotide sequence encoding the affibody of claim 1.

5. The nucleic acid molecule of claim 4, wherein the nucleotide sequence comprises any one of SEQ ID NOS: 9 to 16.

6. A recombinant vector comprising the nucleic acid molecule of claim 4.

7. An isolated host cell comprising the recombinant vector of claim 6.

8. A pharmaceutical composition for preventing or treating cancer, comprising the affibody of claim 1 and a pharmaceutically acceptable carrier.

9. The pharmaceutical composition of claim 8, wherein the cancer is a hematologic cancer or a solid tumor.

10. A protein complex, comprising:
an affibody according to claim 1 that specifically binds to an extracellular domain of CD137; and
an antibody or antigen-binding fragment thereof that specifically binds to a protein on the surface of a cancer cell.

11. The protein complex of claim 10, wherein the protein on the surface of a cancer cell is epidermal growth factor receptor (EGFR), human epidermal growth factor receptor 2 (HER2), or cluster of differentiation 19 (CD19).

12. The protein complex of claim 11, wherein the antibody or antigen-binding fragment thereof that specifically binds to EGFR comprises:
(a) cetuximab; or
(b) a heavy chain variable region comprising a CDR-H1 including the amino acid sequence of SEQ ID NO: 17, a CDR-H2 including the amino acid sequence of SEQ ID NO: 18, and a CDR-H3 including the amino acid sequence of SEQ ID NO: 19; and
a light chain variable region comprising a CDR-L1 including the amino acid sequence of SEQ ID NO: 20, a CDR-L2 including the amino acid sequence of SEQ ID NO: 21, and a CDR-L3 including the amino acid sequence of SEQ ID NO: 22.

13. The protein complex of claim 12, wherein the component (b) comprises: a heavy chain variable region including the amino acid sequence of SEQ ID NO: 23; and a light chain variable region including the amino acid sequence of SEQ ID NO: 24.

14. The protein complex of claim 11, wherein the antibody or antigen-binding fragment thereof that specifically binds to HER2 comprises:
(c) a heavy chain variable region including the amino acid sequence of SEQ ID NO: 28 and a light chain variable region including the amino acid sequence of SEQ ID NO: 29;
(d) trastuzumab; or
(e) a heavy chain variable region comprising a CDR-H1 including the amino acid sequence of SEQ ID NO: 30, a CDR-H2 including the amino acid sequence of SEQ ID NO: 31, and a CDR-H3 including the amino acid sequence of SEQ ID NO: 32; and
a light chain variable region comprising a CDR-L1 including the amino acid sequence of SEQ ID NO: 33, a CDR-L2 including the amino acid sequence of SEQ ID NO: 34, and a CDR-L3 including the amino acid sequence of SEQ ID NO: 35.

15. The protein complex of claim 14, wherein the component (e) comprises a heavy chain variable region including the amino acid sequence of SEQ ID NO: 36 and a light chain variable region including the amino acid sequence of SEQ ID NO: 37.

16. The protein complex of claim 11, wherein the monoclonal antibody or antigen-binding fragment thereof that specifically binds to CD19 comprises:
(f) a heavy chain variable region including the amino acid sequence of SEQ ID NO: 38 and a light chain variable region including the amino acid sequence of SEQ ID NO: 39; or
(g) FMC63.

17. A nucleic acid molecule comprising a nucleotide sequence encoding the protein complex according to any one of claims 10 to 16.

18. A recombinant vector comprising the nucleic acid molecule of claim 17.

19. An isolated host cell comprising the recombinant vector of claim 18.

20. A pharmaceutical composition for preventing or treating cancer, comprising the protein complex according to any one of claims 10 to 16 and a pharmaceutically acceptable carrier.
